# EUROPEAN PATENT APPLICATION

(11) **EP 2 489 368 A1**
(43) Date of publication of application: **22.08.2012**
(21) Application number: 11189162.8
(22) Date of filing: 03.02.2006
(51) Int. Cl.: A61K 39/35, A61P 37/08

(54) **Minor allergen control to increase safety of immunotherapy**

(30) Priority: 03.02.2005 DK 200500177; 03.02.2005 US 649090 P
(62) Divisional of application: 06704631.8
(71) Applicant: Alk-Abelló A/S, 2970 Hørsholm (DK)
(72) Inventor: Hernandez, Domingo Barber, 28037 Madrid (ES); Corrales, Florentino Polo, Decased (ES); Vega, Manuel Lombardero, 28037 Madrid (ES)
(74) Representative: Inspicos A/S

(57) **Abstract**

The invention discloses a method for standardizing olive pollen allergen extract with respect to the contents of major as well as minor allergens. The method comprises determining relative amounts of allergens in a given extract, followed by an adjustment of the contents of major and minor allergens so as to meet predefined limitations. Also disclosed is a method for prepareing anti ole e 9 monoclonal antibodies.

## Description

### FIELD OF THE INVENTION

The present invention relates to the field of immunology and allergology. In particular, the invention relates to the improved safety of allergen vaccines by means of novel production methods that *i.a.* rely on quantifying and adjusting the content of major and minor allergens in immunogenic compositions so that these compositions are adapted to the profile of allergens to which allergic patients are sensitized.

### BACKGROUND OF THE INVENTION

Allergy, *i.e*. type I hypersensitivity, affects millions of people worldwide, and its incidence has increased over the last few years in developed countries, leading to increasing human and economic costs (1). Important clinical manifestations of allergy include asthma, hay fever, eczema, and gastrointestinal disorders. Although allergy in general may not be considered a life-threatening disease, asthma annually causes a significant number of deaths.

Allergy is caused by an inappropriate immunological reaction to foreign, normally non-pathogenic substances. The allergic reaction is specific in the sense that a particular individual is sensitised to (a) certain allergen(s), whereas the individual does not necessarily show an allergic reaction to other substances known to cause allergic disease. The allergic phenotype is characterized by a pronounced inflammation of the mucosa of the target organ and by the presence of allergen specific antibodies of the IgE class in the circulation and on the surface of mast-cells and basophilic granulocytes ("basophils").

IgE-mediated allergic diseases are elicited by proteins, glycoproteins, lipoproteins, and poly-saccharides, called, in general, allergens. Exposure to the allergen can be by inhalation, contact, ingestion, or injection. The most important allergen sources are found among the most prevalent particles of a certain size in environmental air. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, olive and pellitory-of-the-wall in Southern Europe, ragweed in the Eastern and Central United States, and Japanese cedar pollen in Japan. Insects and their products, *i.a*. bee and wasp venoms, and foods each account for approximately 2% of all allergies.

Allergy disease management comprises diagnosis and treatment including prophylactic treatments. Diagnosis of allergy is concerned with the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick tests, blood tests, or provocation tests.

The current treatment for allergic diseases consists primarily in symptomatic relief. Patients are treated with drugs, such as anti-histamines and steroids, which do not suppress the formation of IgE antibodies and often have adverse side effects. As stated in the WHO Position Paper (2), immunotherapy is the only treatment that may affect the natural course of allergic diseases and also may prevent the development of asthma in patients with allergic rhinitis (2). Immunotherapy modulates the immune response in patients throughout the administration of increasing amounts of the appropriate allergenic extract.

Allergenic extracts used therapeutically are crude mixtures of proteins and non-protein components isolated from natural sources. With the discovery in 1966 of the IgE molecule (3, 4), scientific methods were introduced to standardize allergenic extracts in the seventies and eighties (5). These methods were based on the comparison of new production batches to a reference extract, whose potency had been previously measured by skin testing in selected patients. Methods mostly used for the *in vitro* assessment of relative allergenic potency are RAST inhibition or related methods (6). The essential reagent in these methods is a serum pool from a panel of allergic patients, and the potency is measured by comparing the inhibition caused by the extract tested on the binding of specific IgE from the serum pool to a reference extract, coupled to a solid-phase, with the inhibition reached by the reference extract. In this way, the potency of an allergen extract is the sum of the contribution to the allergenic activity from any individual IgE molecule specific for any epitope on any molecule in the allergen extract. Therefore, the potency measures will always depend on the serum pool or patient panel selected, as well as on the reference extract. Moreover, these methods do not provide any information on the qualitative composition of the extracts, that is, the relative concentration of individual allergens. In recent years, with the advent of hybridoma and monoclonal antibody technology, significant progresses have been made in the standardization of allergenic extracts through the determination of major allergen content by monoclonal antibody-based immunoassays. For some species, with only one major allergen, such as *Felis domesticus* (7) or *Plantago lanceolata* (8), a good correlation between the concentration of major allergen and the potency of the extracts has been observed, and even these techniques have allowed not only the definition of the allergen concentration for a determined potency but also the optimum ratio between two major allergens for some species, such as the mites from the genus *Dermatophagoides* (9). These significant advances are greatly acknowledged and major allergen dose is nowadays considered a key parameter in the forecast of the efficacy of immunotherapy (10).

Nevertheless, unexpected adverse effects still constitute a major problem when subjecting allergic patients to immunotherapy with allergen extracts.

Hence, specific allergy vaccination is, in spite of its virtues, not in widespread use, mainly due to the risk of allergic side reactions. Conventional specific allergy vaccination is carried out using multiple subcutaneous immunizations applied over an extended time period. Following each injection the patient must remain under medical attendance for 30 minutes due to the risk of anaphylactic side reactions, which although extremely rare can be life-threatening. In addition, the clinic should be equipped to support emergency treatment. There is no doubt that increasing the safety of the allergy vaccines would facilitate a more widespread use, contributing to solve a major health and economic problem and, at the same time, improving the quality of life of allergic patients.

There is consequently a need to provide allergen extracts with an improved safety.

### SUMMARY OF THE INVENTION

The term "major allergen" is based on a statistical estimation of the prevalence of sensitization to a particular allergen within a general population of patients allergic to a determined allergen extract. Thus, only those allergens that cause sensitization in >50% of patients are called "major allergens" (11). Nevertheless, the present inventors have noted that a very important clinically relevant factor, namely the amount and affinities of IgE antibodies directed against a particular allergen, either major or minor, that an individual patient can produce, is not taken into consideration with the current standardization methods.

Very recently, the present inventors' group have found a significant negative correlation (r=-0.72, p= 0.012, cf. Sastre J et al., 2006, Allergy 61: 206-210) between maximum tolerated dose with latex immunotherapy in healthcare workers and the serum concentration of specific IgE to Hev b 6.01 (prohevein). It is highly probable that this result can be extended to other allergens so that high IgE levels to a particular allergen could imply a lower tolerated dose for this particular allergen in immunotherapy regimes. Current standardization methods based on IgE-immunoassays with serum pools or major allergen immunoassays are able to control in a reasonable way the levels of major allergens and so the safety dose for them. However, there are some uncertainties with low-prevalent allergens, mainly if two conditions are met: high specific IgE levels to the allergen in sensitized patients, and important variability of the allergen content in the raw materials available. Nowadays, all patients sensitive to a given allergenic extract continue to receive the same complex mixture containing all the constituents of the extract only quantitatively controlled regarding the concentration of the more prevalent allergens but ignoring the concentration of statistically minor allergens, even though these could be potentially harmful for some patients who eventually might have high specific IgE levels to these particular allergens. The highest quality allergenic extracts currently in the market are simply subjected to qualitative techniques for quality control, such as SDS-PAGE/Western blot or immunoelectrophoresis, to test the presence of minor allergens.

According to the present invention, this lack of quantitative control on the concentration of minor allergens might be the origin of a proportion of side effects observed when administerring immunotherapy treatments, anaphylaxis being the major risk associated with these treatments. For instance, the present inventors have observed periodical clusters of adverse reactions, apparently linked to determined batches of allergen extracts. However, upon extensive analysis, no association with the quality of the extract, analyzed with the established methods for quality control, can be established, which suggests that adverse reactions might have been caused by a non-controlled parameter, and the present inventors believe that this parameter is the concentration of an allergen normally designated "minor".

The same considerations as above could be applied for the current allergenic extracts used for diagnosis. Thus, those patients who are mostly sensitized to statistically minor allergens might be incorrectly diagnosed if the concentration of the minor allergens they are sensitized to is too low in the extract used, and if too high, they may receive undesirable side-efffects during testing.

The present invention provides means for increasing the safety of allergen extracts intended for clinical use. This present invention hence relates to the definition of allergen sensitisation profiles of allergic patients and the implementation of methods for the production of allergy vaccines adapted to such allergen sensitisation profiles. These production methods preferably include quantitation methods for minor allergens.

The standardization methods currently enforced for the production of allergen vaccines do not take into account the IgE antibodies response of individual patients to the allergens present in the extract. Each allergic patient mounts a unique immune response against the offending allergen source material that depends on genetics, route of exposure and intensity of exposure to the allergen source, among other factors. Consequently, the proteins of the allergenic source to which the patient reacts and the concentration and affinities of IgE antibodies produced are different for each patient. In particular, some patients can strongly react to allergens that do not sensitize the majority of the allergic population, that is, these patients react strongly to minor allergens. Therefore, a safer immunotherapy treatment should be based on the administration of an allergenic vaccine prepared following controlled procedures to ensure an adequate balance of major and minor allergens, minimizing the variations in their concentrations and/or tailoring the allergen concentrations to particular sub-populations wherein some allergens, which are generally considered "minor" function as major allergens. Hence, the production procedures, including selection and mixing, if necessary, of raw materials, have to be established on the basis of the control of the minor allergens that sensitize allergic patients to whom the vaccine is to be administered.

Hence, the present invention provides for 3 important novel realisations: 1) When utilising allergen extracts, it is advantageous to control quantitatively the relative amounts of allergens, major as well as minor, because vast variations may exist between individual batches. 2) Different populations or sub-populations allergic to the same allergenic agent can have markedly different allergenicity profiles, meaning that allergen extracts and allergen compositions can be tailored for each population. 3) The latter realisation also renders possible the preparation of allergen compositions which are not extracts from natural sources, but where the different allergens are balanced with respect to their relative concentrations, so that populations or individuals characterized by allergy to several (major) allergens can be immunized with one type of composition (rich in all major allergens of that population), whereas populations characterized by allergy to a few allergens or even one single allergen can be immunized with a different composition - the latter finding opens up for novel strategies in designing recombinantly produced allergen compositions.

The present invention will e.g. be suitable for improving existing allergen extracts/vaccines e.g. any one of the following products which are marketed by the present assignee, Alk-Abelló:
Allergen vaccines based on extracts are hence already on the market. The present assignee e.g. markets the following allergen vaccine products:
   - Alutard SQ: an alum depot formulation of allergen extract for subcutaneous administration.
   - Aquagen: an aqueous, freeze-dried allergy vaccine, contaning no alum for subcutaneous administration.
   - Pharmalgen: a freeze-dried product co-packaged with a diluent for reconstitution, also for subcutaneous administration.
   - SLITOne: A liquid allergen extract in a single-dose container for oral administration in single doses.
   - Pangramin SLIT: A liquid glycerol formulated allergen extract for oral dropwise administration.

All these products contain allergen extracts which are currently controlled according to existing standards for controlling major allergen content but could be subjected to the methods of the present invention in order to provide increased safety.

### DETAILED DISCLOSURE OF THE INVENTION

### Definitions

In order to provide a clear understanding of the metes and bounds of the present invention, a number of terms used herein are defined in the following:
The term "allergen" refers to any molecule capable of inducing allergy, i.e. IgE mediated reactions upon repeated exposure to an allergen. Examples of naturally occurring allergens include pollen allergens (tree, weed, herb and grass pollen allergens), mite allergens (from e.g. house dust mites and storage mites), insect allergens (inhalant, saliva- and venom origin allergens), animal allergens from e.g. saliva, urine, hair and dander, feathers from e.g. dog, cat, horse, rat, mouse, guinea pig, rabbit, bird etc., fungal or mould allergens and food allergens. Most allergens are proteinaceous, but the present invention is not limited to proteinaceous allergens. The allergen may form part of an allergen extract, or constitute a purified allergen, a modified allergen or a recombinant allergen or a recombinant mutant allergen, or any proteinaceous allergen fragment of more than 30 amino acids.

Examples of naturally occurring allergens include pollen allergens (tree, herb, weed, and grass pollen allergens), insect allergens (inhalant, saliva and venom allergens, e.g. mite allergens, cockroach and midges allergens, hymenoptera venom allergens), urine, animal hair and dander allergens (from e.g. dog, cat, horse, rat, mouse, guinea pig, rabbit etc.), skin and feather allergens (from birds), rubber, worms and food allergens. Important pollen allergens from trees, grasses and herbs are such originating from the taxonomic orders of Fagales, , Lamiales, Pinales and Platanaceae including for example birch (Betula), alder (Alnus), hazel (Corylus), hornbeam (Carpinus) and olive (Olea), cedar (Cryptomeria and Juniperus), Plane tree (Platanus), the order of Poales including for example grasses of the genera Lolium, Phleum, Poa, Cynodon, Dactylis, Holcus, Phalaris, Secale, and Sorghum, the orders of Asterales, Urticales and Rosales including for example herbs of the genera Ambrosia, Artemisia, and Parietaria. Other important inhalation allergens are those from house dust mites of the genus Dermatophagoides and Euroglyphus, storage mite e.g. Lepidoglyphys, Glycyphagus and Tyrophagus, and mites e.g. Blomia, those from cockroaches, midges and fleas e.g. Blatella, Periplaneta, Chironomus and Ctenocepphalides, and those from mammals such as cat (genus Felis), dog (genus Canis), cow (genus Bos), horse (genus Equus), rat (genus Rattus), mice (genus Mus) and guinea pig (genus Carvia), venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are, for example, those originating from the genera Alternaria and Cladosporium.

Examples of allergens include Bet v 1, Bet v 2, Aln g 1, Cor a 1 and Car b 1, Cas s 1, Cas s 5, Quea a 1, Cry j 1, Cry j 2 , Cup a 1, Cup s 1, Jun a 1, Jun a 2, Jun a 3, Jun o 4, Jun s 1, Jun v 1, Ole e 1 , Ole e 2, , Ole e 5, Ole e 8, Ole e 9, Syr v 1, Lig v 1, Pia l 1, Pia a 2, Pia a 3, Amb a 1, Amb a 2, Amb a 3, Amb a 5, Amb a 6, Amb a 7, Amb t 5, Art v 1, Art v 2, Art v 3, Art v 4, Par j 1 , Par j 2, Par j 3, Par o 1, Sal k 1, Ave e 1, Cyn d 1, Cyn d 7, Cyn d 12, Dac g 1, Dag g 2, Dag g 3, Fes p 1, Fes p 4, Hol I 1, Lol p 1. Lol p 5, Lol p 3, Lol p 2, Pha a 1, Pas n 1, Phl p 1, Phl p 2, Phl p 3, Phl p 4, Phl p 5, Phl p 6, Poa p 1, Poa p 5, Sec c 1, Sec c 5, Sor h 1, Der f 1 , Der f 2, Der f 3, Der f 7, Der f, 10, Der f 11, Der f 14, Der p 1, Der p 2, , Der p 3, Der p 4, Der p 5, Der p 6, Der p 7, Der p 8, Der p 10, Der m 1, Eur m 2, Eur m 14, Gly d 1, Gly d 2, Lep d 2, Lep d 5, Lep d 7, Lep d 10, Lep d 13, Blo t 1, Blo t 3, Blo t 6, Blo t 10, Tyr p 2, Bla g 1, Bla g 2, Bla g 4, Bla g 5, Bla g 6, Per a 1, Per a 3, Per a 7, Per f 1, Fel d 1, Fel d 2, Can f 1, Can f 2 , Can f 3, Bos d 2, Bos d 4, Bos d 8, Equ c 1, Equ c 2, Equ c 3 , M us m 1, Rat n 1, Cav p1, Cav p 2, Apis m 1, Api m 2 ,Apis m 4, Ves v 1, Ves v 2, Ves v 5, Dol m 1, Dol m 2, Dol m 5, Pol a 1, Pol a 2, Pol a 5, Sol i 1, Sol i 2, Sol i 3 and Sol i 4, Alt a 1, Alt a 2, Alt a 3, Alt a 6, Cla h 1, Cha I 2, Cha I 3, Asp f 1, Asp f 2, Asp f 3, , Mal d 1, Gly m 1, Gly m 2, Gly m 3, Ara h 1, Ara h 2, Ara h 3, Ara h 4, Ara h 5, see further the web-site "The official 'List of Allergens"'. (Allergen Nomenclature. International Union of Immunological Societies, Allergen Nomenclature Sub-Committee) http://www.allergen.org/ for a comprehensive list of identified single allergens.

In a preferred embodiment of the invention the allergen is tree pollen allergen or grass pollen allergen or a dust mite allergen or a ragweed allergen or a cedar pollen or a cat allergen or a cockroach allergen. Another preferred allergen is an olive tree pollen allergen.

The term "allergen sensitisation profile" is in the present context intended to denote the pattern of IgE reactivity which is exhibited by an individual or a population, as the case may be, against individual allergens derived from an allergenic agent. In an individual, the profile provides information solely of IgE reactivity in that individual against a certain allergen, whereas in a population, the profile will also provide a statistical readout which includes a further dimension that provide information of the percentage of individuals in the population who reacts to a given allergen.

The expression "allergen extract" as used therein refers to an extract obtained by extraction of a biological allergen source material as generally described in "Allergenic extracts", H. Ipsen et al, chapter 20 in Allergy, principle and practise (Ed. S. Manning) 1993, Mosby-Year Book, St. Louis. Such extracts may be obtained by aqueous extraction of water soluble material followed by purification steps like filtration to obtain the solution i.e. the extract. The extract may then be subjected to further purification and/or processing like freeze-drying removing substantially all the water. Generally, an allergen extract comprises a mixture of proteins and other molecules.

Allergen proteins are often classified as a "major allergen", or a "minor allergen". An allergen extract generally comprises both major and minor allergens. Major allergens will generally constitute approximately 5-15% of an average allergen extract, more often about 10%. Classification of an allergen is based on an assessment of the clinical importance of the particular allergen and is given below. Examples of important major allergens found in an extract include grass group 1 and 5 and 6 allergens (e.g. Phl p 1, 5, and 6), dust mite group 1 and 2 allergens (e.g. Der p 1 , Der p 2), tree pollen allergen 1 (e.g. Bet v 1, Ole e 1), cedar pollen allergen 1 and 2 (e.g. Cry j 1, Cry j 2), ragweed pollen 1 and 2 (Amb a 1, Amb a 2), cat allergen 1 (i.e. Fel d1), cockroach allergens (e.g. Per a 1 or Per f 1). The average allergic person will be sensitised to and react to one or more major allergens and further may also be sensitised and react to one or more minor allergens.

Amounts of allergen extract referred to herein refers to the dry matter content of such allergen extracts.

Preferably the water content of the dry matter does not exceed 10%, more preferably 5% by weight.

The expression "biological allergen source material" as used herein refers to any biological material comprising one or more allergens. Examples of such materials are acarids PMB (Pure Mite Body) or WMC (Whole Mite Culture), defatted or non-defatted pollens from e.g. grasses, herbs, weeds and trees, animal hair and dander, pelt, fungi mycelia and spores, insect bodies, venom or saliva and foods. The term "biological allergen source material" is used interchangeably herein with the term "allergenic agent".

A "major allergen" is an allergen as defined above, which in a given population causes sensitization in more than 50% of allergic patients that are allergic to the allergenic agent from which the allergen is derived. The classification of an allergen as a major allergen can be subject to several tests. An allergen is commonly classified as a major allergen if at least 25% of the patients show strong IgE binding (score 3) and at least 50% of the patients show moderate binding (score 2), the binding being determined by an CRIE (Crossed Radio Immune Electrophoresis) (CRIE Strong binding, i.e. visible IgE-binding on an X-ray film after one day; CRIE Moderate binding, i.e. binding after 3 days; CRIE Weak binding, i.e. binding after 10 days). All other allergens are consequently termed "minor allergens".

Other methods may also be used in determining the IgE binding of for instance IgE-blots.

Generally recognized major allergens include grass group 1 allergens, e.g. phl p 1, lol p 1 , sor h 1, dac g 1, cyn d 1, hol I 1, pha a 1, grass group 2/3 allergen e.g. phl p 2/3, lol p 2/3, grass group 5 allergen e.g. phl p 5, lol p 5, dac g 5, poa p 5, grass group 6 allergen e.g. phl p 6, poa p 6, tree pollen group 1 allergen e.g. bet v 1, aln g 1, cor a 1, car b 1, ole e 1, mite group 1 allergen e.g. der p 1, der f 1, eur m 1, mite group 2 allergen e.g. der p 2, der f 2, eur m 2, Blo t 1, cat allergen e.g. fel d 1, cedar group 1 and group 2 allergen e.g. cry j 1, cry j 2, short or giant ragweed pollen allergen e.g. amb a 1, amb a 2, amb t 1, amb t 2, other weed allergens e.g. par j 1, par o 1, par j 2, insect allergens e.g. per a 1 bla g 1, per f 1, ves v 1 ves v 5, pol a 1, dol m 1.

As will be clear from the present description, minor allergens may, if one exclusively considers a subset of an allergic population, constitute "major allergens" (since some individuals react with a minor allergen, and these individuals logically constitute a subset of a larger allergic population). This underscores the fact that the terms "major allergen" and "minor allergens" are relative and depend on the allergic population one is studying. However, in order for an allergen to be regarded as a major allergen within the present disclosure and claims, the population reacting with the "major allergen" has to have a certain size, typically at least 30 individuals, and be distinguishable from other patients in a larger allergic population with respect to at least one further common demographic parameter that renders it possible to readily identify such a population - such a parameter can be geographical (all patients in the population live in a defined geographical area), genetic (all patients in the population have the same origin or share a common genetic or phenotypic marker), environmental (e.g.: all individuals share a common exposure history), or any other demographic parameter.

A "standardized allergen extract" herein denotes an extract of allergens from a particular allergenic agent, where the allergen components in the extract are adjusted in order to obtain an adequate balance between major and minor allergens as well as adequate and safe amount of the individual allergens. In particular, the standardized allergen extracts of the invention are those, where the extract is matched against the allergen sensitization profile of a given population so as to minimize adverse effects when immunizing members of such a population.

The term "immunogenically effective amount" shall mean a dose of allergen(s) which, when taken once or repeatedly in a monodose or in incremental doses results in, for example, an adaptive immune response and thus serves as means to desensitise allergic patients. Preferably, the term shall mean the amount of allergen in each dosage form necessary to induce an adaptive immune response after repeated administration of said dosage forms in accordance with a treatment regimen (over a period ranging from a few applications to at least one daily application over several months). Preferably desensitization includes the alleviation of allergic symptoms upon administration of the dose. Clinical allergy symptoms include rhinitis, conjunctivitis, asthma, urticaria, eczema, which includes reactions in the skin, eyes, nose, upper and lower airways with common symptoms such as redness and itching of eyes and nose, itching and runny nose, coaching, weezing, shortness of breathe, itching, and swelling of tissue.

A "maximum acceptable" amount or dose is the dose the skilled practitioner will regard as the maximum safe dose of a particular substance to be administered so a patient. For an allergen, the amount will in an allergenic be quite small (in the lower microgram range), but sensitisation will usually begin at lower doses which for safety reasons are regarded as the maximum acceptable.

The term "single dosage form" is intended to designate a one-dose formulation derived/isolated from an allergen extract prepared according to the present invention. The dosage form can be obtained by concentrating the extract or by diluting the extract and by formulating the single dosage form according to any convenient dosage form known in the art. This means that the total concentration of allergens in the single dosage form can be different from the concentration in the extract, but that the ratios between amounts of the individual allergens in the extract are essentially identical to the ratios between the amounts of the same individual allergens in the single dosage form.

Biological allergen source materials/allergenic agents may comprise contaminating materials, such as foreign pollen and plant and flower debris for an allergen pollen source material.

The degree of contamination should be minimised. Preferably, the content of contaminants should not exceed 10% (W/W) of the biological source material.

Normally an allergen extract contains at least 10% protein of the dry matter content of the allergen extract as determined in a standard protein assay such as BCA or Lowry and the remainder consists of other "non-protein material" which may be components such as lipids, carbohydrates, or bound water which originate from the biological allergen source.

An allergen extract may be formulated and stored in form of a freeze-dried material obtainable by freeze-drying a liquid allergen extract at a pressure of below 800 micro bar and for a period of up till 100 hours removing the water.

### Brief discussion of the findings leading to the invention

The present inventors have observed that allergic adverse reactions tend to be clustered in time and, in some cases, also in space, being confined to a determined geographical area. Usually, investigations on the causes of the adverse reactions have been focused on the investigation of the composition of particular batches of the allergen vaccines and, commonly, these batches fulfil the quality control specifications established.

Therefore, the present inventors aimed to search for alternative explanations for these adverse reactions. First, focus was put on the variability of allergenic extracts and, second, on the different response of patients to statistically minor allergens. In the examples that illustrate this invention, a surprising and extreme variability both in the composition of allergen extracts and the IgE antibody responses of the patients is observed.

It is worth mentioning that most of the allergenic extracts presented in the examples could have been commercialized fulfilling the quality controls, established according to the current criteria of standardization, *i.e*. total allergenic potency and major allergen content. Moreover, the present inventors have detected that a number of patients exhibit a very strong IgE response to allergens normally defined as minor allergens.

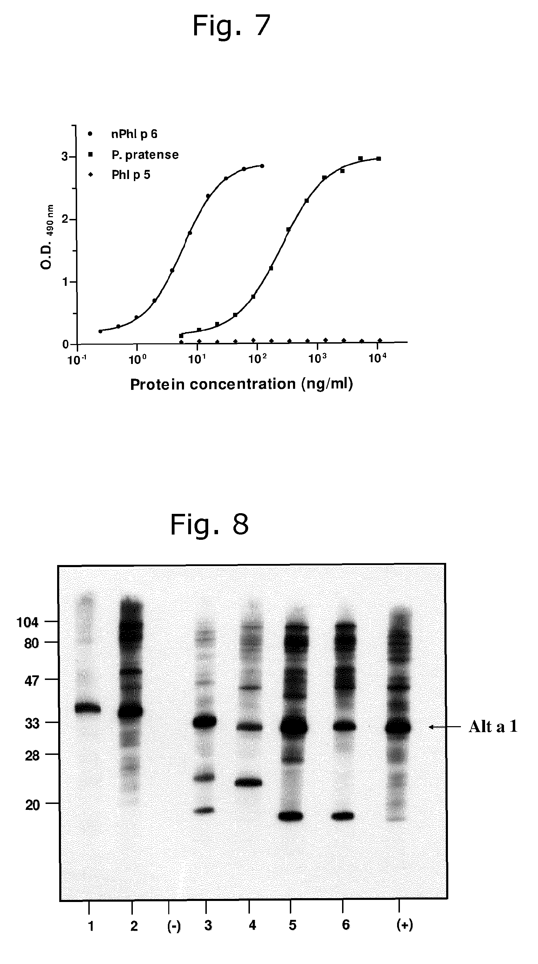

This is clearly seen in the Fig. 8 related to the example on *Alternaria* allergens shown below. In some cases, as in the example relating to sensitization to olive pollen, evidence is shown that patients exposed to different atmospheric pollen levels display different sensitization profiles. Thus, patients living in geographical areas where olive trees are extensively grown exhibit high IgE levels to an allergen (Ole e 9) that is rarely recognized by the IgE from patients living outside these areas. This allergen, according to the definition of major and minor allergens, should be considered as a minor allergen, since it is not recognized by more than 50% of patients sensitized to olive tree pollen. However, in those particular areas where olive trees are grown extensively, this allergen might be considered as a major allergen, since it is recognized by a high proportion of patients. Moreover, the present inventors have found that all the patients sensitized to Ole e 9 are also sensitized to the "traditional" major olive tree allergen Ole e 1 and that the median value of IgE levels to Ole e 9 is high. It seems evident that treatment of highly exposed allergic patients with an allergen vaccine in which the content of Ole e 9 were not adequately controlled could imply an elevated risk of anaphylactic reactions.

With the purpose of establishing a production method for olive tree allergenic products with a known content of this allergen, the present inventors have developed an ELISA to quantify Ole e 9. Testing of different batches of olive tree pollen using this ELISA disclosed an extreme and unexpected variability in the content of this allergen, and also a high variability in the ratio between Ole e 1 and Ole e 9.

It is worth mentioning that, with the current standardization methods, batches of allergen vaccines with 160-fold variations in the concentration of Ole e 9 could be released, implying a high risk of severe adverse reactions. Consequently, by using the present methodology, the variability of allergen extracts can be significantly reduced, and the balance between major and minor allergens can be maintained within adequate limits that minimize the risk of adverse reactions.

The technique developed not only can be applied for in-process controls and for analysis of intermediate and final products but also, and more important, for the selection of raw materials. The biological origin of allergen source materials, such as pollens, moulds, mites, etc., confer them an inherent variability. In particular, pollens show a high batch-to-batch variability, depending on the varieties, geographical location, and pollen season. This means that it might be highly improbable to find a single pollen batch with an adequate balance between major and minor allergens, and to ensure the supply of similar batches for production of consistent batches of allergen vaccines. However, with access to the findings herein and the immunoassay developed, mixes from several pollen batches, previously analyzed, can be prepared to obtain an adequate ratio between major and minor allergens.

An other aspect related to minor allergens that has been considered in the present invention is the possibility that some minor allergens could cross-react with major allergens from the same source. In such cases, IgE-cross-reacting epitopes in both the major and the minor allergen would have additive effects on the binding of IgE antibodies from patients. Therefore, although the major allergen content were quantified in the extract, the presence of a not controlled, high content of the minor allergen might imply an increased risk of adverse reactions in patients sensitized to cross-reacting epitopes. An example of this situation is given in the examples. The present inventors have developed an immunoassay to quantify a minor allergen in pollen of *Phleum pratense* (Phl p 6) that cross-reacts with the major allergen Phl p 5. The analysis of several batches of P. pratense shows a significant variability of this minor allergen that should be taken into account when preparing allergen vaccines.

In summary, statistically minor allergens are very relevant allergens for a fraction of allergic patients, and the combination of a determined allergen sensitization profile with the administration of an allergy vaccine with a high content of the involved minor allergens could lead to severe adverse reactions. Consequently, to increase the safety of allergen vaccines, it is important to minimize the variations of minor allergen content. This can be achieved by using methods to control the content of minor allergens.

### Aspects of the present invention relating to preparation of extracts

In a first aspect the present invention relates to a method for the preparation of a standardized extract of an allergenic agent, comprising
- obtaining an allergen containing extract derived from said allergenic agent,
- determining the relative concentrations of major and minor allergens in said allergen containing extract, wherein said major and minor allergens are defined according to the allergen sensitisation profiles obtained from a population allergic to the allergenic agent,
- adjusting the concentrations of said major and minor allergens to obtain said standardized extract so that 1a) one single dosage form isolated from said standardized extract comprises a total amount of major allergens which does not exceed the maximum acceptable amount of any one single major allergen of said allergenic agent and/or 1b) the concentrations of major and minor allergens in the standardized allergen extract are controlled quantitatively, and 2) that one single dosage form isolated from said standardized extract comprises an immunogenically effective amount of each of said major and optionally minor allergens.

Hence, this aspect of the invention relates to the preparation of allergen extracts that match a certain allergic population with respect to the relative amounts of allergens therein. As discussed above, the present invention has rendered possible the tailoring of such allergen extracts for particular allergic populations, whereby the risk of adverse effects in the immunization process is minimized. Notably, it is ensured that the total concentration of major allergens is levelled so that the risk of adverse effects imposed by other major and minor allergens as well as cross-reacting major allergens is minimized. To the best of the present inventors' knowledge, it has never before been attempted or suggested to control allergen content in extracts in such a way.

In a second aspect, the present invention pertains to a method for the preparation of a set comprising at least 2 mutually distinct standardized extracts of an allergenic agent, said standardized extracts each being adapted for immunization of different allergic populations that are allergic to the allergenic agent, comprising
- obtaining an allergen containing extract derived from said allergenic agent,
- determining the relative concentrations of allergens in said allergen containing extract,
- on the basis of the determination, preparing a member of said set for each of at least 2 different populations allergic to the allergenic agent, each of said at least 2 different populations defining different major allergens, wherein the concentrations of said allergens in each member are adjusted so that 1a) one single dosage form isolated from a member comprises a total amount of major allergens not exceeding the maximum acceptable amount of any one single major allergen of said allergenic agent and/or 1b) the concentrations of major and minor allergens in the standardized allergen extract are controlled quantitatively, and 2) that one single dosage form taken from said member comprises an immunogenically effective amount of each of said major and optionally minor allergens.

Hence, this aspect provides for the possibility of preparing differently composed allergen extracts which are suited for immunization of different allergic populations that are allergic to the same allergenic agent, thereby 1) optimising treatment of the population (or individuals) that are allergic to only one single allergen and 2) minimising the risk of adverse effects in patients allergic to 2 or more allergens from the allergenic agent.

In a third aspect, the present invention relates to a method for preparation of standardized allergen extracts from an allergenic agent, the method comprising obtaining allergen extracts derived from the allergenic agent, determining the concentrations of major and minor allergens in the individual extracts, and if necessary adjusting the relative amounts of major and minor allergens in the extracts to obtain standardized allergen extracts where the relative amounts of major and minor allergens are within predefined boundaries so as to allow isolation, from the standardized allergen extracts, of single dosage forms wherein 1) the total amount of major allergens do not exceed the maximum acceptable amount of any one single major allergen from said allergenic agent and/or the concentrations of major and minor allergens are controlled quantitatively, and 2) the major and optionally minor allergens are present in immunologically effective amounts.

This 3^{rd} aspect provides for a more simple part of the invention, where a given allergen extract is tested for its composition - if the extract fulfils the criteria of allergen content vis-à-vis the relevant allergic population, the allergen extract "passes the test" and is not modified, whereas an allergen extract not fulfilling the criteria will be subjected to an adjustment in allergen content as described in detail herein.

A particular aspect of the invention is a method for the preparation of a standardized extract of an allergenic agent, comprising
- obtaining an allergen containing extract from the allergenic agent,
- determining the relative concentrations of allergens in said allergen containing extract, wherein said allergens are defined by the allergen sensitisation profile of an allergic subject,
- adjusting the concentrations of said allergens so that 1) one single dosage form of said extract comprises a total amount of allergens which does not exceed the maximum acceptable amount of any one single allergen of said allergenic agent and/or the concentrations of all allergens in the standardized allergen extract are controlled quantitatively, and 2) that one single dosage form of said extract comprises an immunogenically effective amount of each of said allergens.

Hence, this aspect, which is based on the inventive findings with respect to the possibility of vast variations in allergen content in extracts of allergens, provides for the most "individualized" form of the present invention. In this particular aspect, the terms "major" and "minor" allergens are not of relevance, but nevertheless it is of importance to adjust the allergen content of a given extract in order not to endanger the subject who is going to be ultimately immunized.

Typically, the inventive methods for preparation of allergen extracts will be repeated over time in order to produce lines of extracts that are similar in composition, so as to allow uniform treatment regimens of patients over time. Hence, the inventive methods also entail that multiple extracts are prepared. In this case, allergen concentration (e.g. major allergen and minor allergen concentration) in any one of said multiple extracts has a variation of at most 50% compared to any other one of said multiple extracts. However, much smaller variations are preferred, such as at most 40%, at most 30%, at most 25%, at most 15%, at most 5%, at most 2% and at most 1%.

It will, however be understood that *single* allergens may be allowed to vary considerably more than the variations allowed for major and/or minor allergens in general. For example, according to the Monograph on Allergen Products of the European Pharmacopoeia, a variation in the concentration of an allergen between 50% and 200% of the nominal value is admitted. For instance, if the nominal value for Ole e 1 content in Olea europaea extracts is 60 µg/ml, the variation allowed would be between 30 and 120 µg/ml.

Hence, when preparing multiple extracts it is according to the invention acceptable that the concentration of any one major allergen and/or of any minor allergen in any one of the multiple extracts does not exceed the concentration in any other one of the multiple extracts by a factor of more than 6. The factor may of course be lower, so that it does not exceed 5 or 4 (which complies with the above limit set forth in the Monograph) or 3 or 2.

It will be clear to the skilled person, that the allergen content in the standardized extracts can be adjusted by means of several methods. The simplest is to determine the relative concentrations of relevant allergens in a large number of allergen extracts obtained by means known by the skilled person, and then using these different batches in order to prepare adequately balanced mixtures of 2 or more batches of allergenic extracts so as to obtain an adequately balanced standardized extract.

Alternatively, certain allergens can be removed by purification methods known in the art (most conveniently antibody affinity purification due to its high specificity), and in the rare cases where the balance of allergens is adequate except for too low concentration of one or a few allergens, the extracts can be spiked with the relevant allergens.

The standardized extracts obtained by means of the present invention can be both concentrated (even dried) or diluted, but according to the present invention they are still regarded to be "standardized extracts" as long as the relative concentrations of allergens are left unaltered.

### Other allergen compositions

It is believed by the present inventors that the concept underlying the present invention, namely the fact that "major" and "minor" allergens are population dependent. Hence, other art-recognized methods of preparing allergen vaccines which rely on e.g. recombinant products, may suffer some of the same problems as do the traditional allergen extract, because they are not composed so as to be optimized for a given, well-defined population.

Hence, the invention also relates to a method for preparing a standardized allergen composition, the method comprising determining the allergen sensitisation profile of a population so as to identify major allergens and minor allergens reactive in said population, subsequently admixing the major and minor allergens thus identified, so that 1a) the concentrations of said major and minor allergens in one single dosage form isolated from said standardized allergen composition comprises a total amount of major allergens which does not exceed the maximum acceptable amount of any one single major allergen of said allergenic agent and/or 1b) the concentrations of major and minor allergens in the standardized allergen composition are controlled quantitatively, and 2) that one single dosage form isolated from said allergen composition comprises an immunogenically effective amount of each of said major and optionally minor allergens. The standardized allergen composition may be subsequently concentrated or diluted.

The allergens in such a composition are preferably isolated from a natural source and/or recombinantly produced and/or prepared by means of synthesis - the latter option is for proteinaceous allergens especially suited for small allergens which can be synthesized by the methods of liquid-phase or solid-phase peptide synthesis.

### Preparing pharmaceutical preparations (vaccines)

The bulk manufacture of the allergen extracts subject to the methods of the current invention are well known in the art see e.g. Allergenic extracts (Allergy Principles and Practice, Chapter 20, Ipsen et al, Mosby-Year Book, 1993). Extracts may be stored depending on the overall requirements, most often either in the form of chilled or frozen extract or dried or freeze-dried extract see e.g. WO2005/058474.

The allergenic extracts prepared according to the present invention are suitable as pharmaceuticals and vaccines in their own right but may also be manipulated further (by means of methods known in the art) to prepare specific pharmaceutical preparations, cf. the discussion below concerning formulation of allergens for the purpose of preparing pharmaceuticals.

Hence, the present invention also relates to a method for the preparation of a pharmaceutical composition for inducing tolerance to an allergen, the method comprising preparing a standardized extract or composition according to a method of the invention and subsequently formulating the thus obtained standardized extract together with a pharmaceutically and immunologically acceptable carrier, vehicle or diluent. Here it should be remembered that the standardized extract may be both diluted and concentrated (such as freeze dried), so it is possible to prepare compositions with any relevant concentration and amount of allergen.

Preparation of vaccines which contain peptide sequences and polypeptides as active ingredients is generally well understood in the art, as exemplified by U.S. Patents 4,608,251; 4,601,903; 4,599,231; 4,599,230; 4,596,792; and 4,578,770, PCT/DK2005/000601 and WO 2004/075875, all incorporated herein by reference. Typically, such vaccines are prepared as injectables either as liquid solutions or suspensions; solid forms suitable for solution in, or suspension in, liquid prior to injection may also be prepared. The preparation may also be emulsified. The vaccine may further also be prepared as a liquid for topical e.g. mucosal administration. The active immunogenic ingredient is often mixed with excipients which are pharmaceutically acceptable and compatible with the active ingredient prior to or in the course of final formulation of the vaccine. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines; cf. the detailed discussion of adjuvants below.

Vaccines may further be prepared for solid dosage forms such as tablets, compresed or non-compressed, capsules and lozenges. The vaccines may be prepared as sold intermediates well known in the art such as granules, micro- or nano particles or powders for the further formulation in the solid dosage forms. Vaccines in solid dosage are describe in e.g. WO 2004/075875 and in particular in fast dispersing solid exemplified in US 6,709,669 EP 1 024 824, EP 1 154 757, WO 2005/120464, and WO 2004/047794, all incorporated herein by reference.

The vaccines are conventionally administered parenterally, by injection, for example, either subcutaneously, intracutaneously, intradermally, subdermally or intramuscularly. Additional formulations which are suitable for other modes of administration include, oromucosal i.e. oral, buccal, sublinqual, gastrointestinal formulations, suppositories and, in some cases intraperitoneal, intravaginal, anal, epidural, spinal, and intracranial formulations. For suppositories, traditional binders and carriers may include, for example, polyalkalene glycols or triglycerides; such suppositories may be formed from mixtures containing the active ingredient in the range of 0.5% to 10%, preferably 1-2%. Oral and oromucosal formulations include such normally employed excipients as, for example, pharmaceutical grades of gelatine (of mammal or non-mammal origin), mannitol, lactose, starch, magnesium stearate, sodium saccharine, cellulose, magnesium carbonate, and the like. Non-parenteral compositions take the form of solutions, suspensions, tablets, non-compressed fast dispersing formulations e.g. lozenges, pills, capsules, sustained release formulations or powders and for allergens the content of the active ingredients may be as little as 1‰, most often in the range of 0.01 -25 %, preferably 0.1 %- 10%.

The allergens may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like. Chemically modified, e.g. glutaraldehyde modified, allergens are also envisaged. Sometimes it can be beneficial to let the vaccine also comprise an adjuvant substance, especially if the adjuvant is capable of stimulating the correct subset of T-cells in order to switch the allergic IgE response to an IgG response.

Various methods of achieving adjuvant effect for the vaccine are known. General principles and methods are detailed in "The Theory and Practical Application of Adjuvants", 1995, Duncan E.S. Stewart-Tull (ed.), John Wiley & Sons Ltd, ISBN 0-471-95170-6, and also in "Vaccines: New Generation Immunological Adjuvants", 1995, Gregoriadis G et al. (eds.), Plenum Press, New York, ISBN 0-306-45283-9, both of which are hereby incorporated by reference herein.

Non-limiting examples of suitable adjuvants are selected from the group consisting of an immune targeting adjuvant; an immune modulating adjuvant such as a toxin, a cytokine, and a mycobacterial derivative; an oil formulation; a polymer; a micelle forming adjuvant; a saponin; a particle; DDA; aluminium adjuvants; DNA adjuvants, Cpg adjuvants; γ-inulin; Glycolipid adjuvants, non-pathogenic bacteria and an encapsulating adjuvant

The application of adjuvants include use of agents such as aluminium hydroxide or phosphate (alum), commonly used as 0.05 to 0.1 percent solution in buffered saline, admixture with synthetic polymers of sugars (*e.g.* Carbopol®) used as 0.25 percent solution, aggregation of the protein in the vaccine by heat treatment with temperatures ranging between 70° to 101°C for 30 second to 2 minute periods respectively and also aggregation by means of cross-linking agents are possible. Aggregation by reactivation with pepsin treated antibodies (Fab fragments) to albumin, mixture with bacterial cells such as *C. parvum* or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. Admixture with oils such as squalene and IFA is also preferred.

Liposome formulations are also known to confer adjuvant effects, and therefore liposome adjuvants are preferred according to the invention. Also immunostimulating complex matrix type (ISCOM® matrix) adjuvants are preferred choices according to the invention, especially since it has been shown that this type of adjuvants are capable of up-regulating MHC Class II expression by APCs. An ISCOM® matrix consists of (optionally fractionated) saponins (triter-penoids) from *Quillaja saponaria,* cholesterol, and phospholipid. When admixed with the immunogenic protein, the resulting particulate formulation is what is known as an ISCOM particle where the saponin constitutes 60-70% w/w, the cholesterol and phospholipid 10-15% w/w, and the protein 10-15% w/w. Details relating to composition and use of immunostimulating complexes can *e.g.* be found in the above-mentioned text-books dealing with adjuvants, but also Morein B et al., 1995, Clin. Immunother. 3: 461-475 as well as Barr IG and Mitchell GF, 1996, Immunol. and Cell Biol. 74: 8-25 (both incorporated by reference herein) provide useful instructions for the preparation of complete immunostimulating complexes.

Other possibilities involve the use of the targeting and immune modulating substances (*i.a.* cytokines). In this connection, also synthetic inducers of cytokines like poly I:C are possibilities.

Suitable mycobacterial derivatives are selected from the group consisting of muramyl dipeptide, complete Freund's adjuvant, RIBI, and a diester of trehalose such as TDM and TDE.

Suitable DNA adjuvant include ImmuneStimulatory Sequences (e.g. CpG motif containg DNA sequences) and similar components

Suitable Glycolipid adjuvant include LPS (Lipo-Poly-Saccharides) compounds and composistions and MPL (Mono Phosphoryl Lipid A) and derivatives thereof such described in US 4,803,070, US 4,806,352, US 4,866,034, US 4,,987,237, US 5,762,943, EP 0 729 473 and WO 01/46127

Suitable immune targeting adjuvants are selected from the group consisting of e.g. CD40 ligand and CD40 antibodies or specifically binding fragments thereof, mannose, a Fab fragment, and CTLA-4.

Suitable polymer adjuvants are selected from the group consisting of a carbohydrate such as dextran, PEG, starch, mannan, and mannose; a plastic polymer; and latex such as latex beads.

Microparticle and nanoparticle formulations of vaccines has been shown in many cases to increase the immunogenicity of protein antigens and is therefore another preferred embodiment of the invention. Microparticles are made either as co-formulations of antigen with a polymer, a lipid, a carbohydrate or other molecules suitable for making the particles or the microparticles can be homogeneous particles consisting of only the antigen itself.

Examples of polymer based micro- and nanoparticles are PLGA and PVP based particles (Gupta RK *et al.,* 1998) where the polymer and the antigen are condensed into a solid particle. Lipid based particles can be made as micelles of the lipid (so-called liposomes) entrapping the antigen within the micelle (Pietrobon PJ, 1995). Carbohydrate based particles are typically made of a suitable degradable carbohydrate such as starch or chitosan. The carbohydrate and the antigen are mixed and condensed into particles in a process similar to the one used for polymer particles (Kas HS *et al.,* 1997). Also calciumphosphate particles as described in US 5,219,577 and WO03/051394 are examples.

Particles consisting only of the allergens can be made by various spraying and freeze-drying techniques. Especially suited for the purposes of the present invention is the super critical fluid technology that is used to make very uniform particles of controlled size (York P, 1999 & Shekunov B *et al.,* 1999).

### Controlling the content of allergen components in the extracts

It is preferred that the concentration of both major and minor allergens in the standardized allergen extracts or compositions used and produced according to the present invention is controlled quantitatively to be within predefined limits. As already discussed above, the so-called minor allergens can, if using current methods for extract production, vary considerably, so therefore it is of interest to define the relative concentrations between major and minor allergens which are clinically acceptable and allow for production of clinically acceptable pharmaceuticals. Typically, the concentration of each minor allergen in the standardized extract or composition should be adjusted to be lower than the concentration of the concentration of the major allergen of lowest concentration in the standardized extract or composition, but for certain minor allergens, it is more important that the minor allergens have a *defined* concentration rather than a concentration which is higher or lower than that of the major allergens. In fact, there may be some cases in which the minor allergens are more abundant than the major allergens without this constituting a de facto problem, as long as the concentration of such a minor allergen is controlled. For instance, in cat dander extracts, the albumin, normally a minor allergen, is far more abundant than the major allergen Fel d 1, but in most populations this will not constitute a clinical problem.

Nevertheless, it is preferred, that the weight or molar ratio between the most abundant minor allergen and any major allergen in a single dosage form does not exceed 1:50., but lower amounts of minor allergens are often desired, so the weight or molar ratio between any minor allergen and the most abundant major allergen should not exceed 1:100 or even 1:200.

Likewise, the weight or molar ratio between the most abundant major allergen in the standardized extract or composition and any other major allergen in a single dosage form should not exceed 30: 1, but it is desired that major allergens are present in as abundant fractions as possible. Hence, it is preferred that the weight or molar ratio between the most abundant and any other major allergen does not exceed 15: 1. It is more preferred that the weight or molar ratio between the most abundant and any other major allergen does not exceed 10: 1, and even that it does not exceed 5:1 1 or even 2.5:1.

At any rate, when preparing or using a standardized allergen extract or composition, it is preferred that it comprises all major allergens from said allergenic agent. This ought to ensure the most effective induction of tolerance against the allergenic agent.

### Standardizing allergenic extracts

In the field of allergy extracts, there is no international accepted standardization method. A number of different units of extract strength *i.e.* bio-potency exist. The methods employed and the units used normally measure the allergen content and biological activity. Examples hereof are SQ-Units (Standardised Quality units), BAU (Biological Allergen Units), BU (biological units), UM (Units of Mass), IU (International Units) and IR (Index of Reactivity). Hence, extracts need to be standardized against well-defined extracts in order to determine their potency in SQ units or any of the above mentioned units. The subject matter is dealt with in "Allergenic extracts", H. Ipsen et al, chapter 20 in Allergy, principle and practise (Ed. S. Manning) 1993, Mosby-Year Book, St. Louis and Løwenstein H. (1980) Arb Paul Ehrlich Inst 75:122. Further, guidance to the normally applied, acceptable tests measuring biopotency of allergens are found e.g. in Note for Guidance on Allergen Product; The European Agency for the Evaluation of Medicinal Product, CPMP_BWP_243_96, London, 1996.

Several laboratory tests are available for characterizing an allergen. The most widely used techniques are sodium dodecyl sulphate polyacrylamide gel electrophoresis (SDS-PAGE), isoelectric focusing (IEF), crossed immunoelectrophoresis (CIE) and Rocket Immuno Electrophoresis (RIE). The quantification of individual allergens may be performed by a variety of quantitative immunoelectrophoretic techniques (QIE), Radial Immune Diffusion (RIE) or by enzyme-linked immunosorbent assays (ELISA). The determination of total allergenic activity is most frequently performed by radio allergosorbent test (RAST), Magic Lite assay (LIA) or related techniques. ELISA-based techniques may also be used.

The allergen content of a single dosage form according to the invention can thus be determined by routine immune assays such as CIE (Cross Immune Electrophoresis), RIE (Radio Immune Electrophoresis) and SDS-PAGE (Sodium Dodecyl Sulphate Poly Acrylamide Gel Electrophoresis) and immune assays such as ELISA and Magic Like Specific IgE assay (LIA) against extract components such as major and/or minor allergens.

The bio-potency, i.e. the *in vivo* allergenic activity, of a given extract depends on a number of factors, the most important being the content of major allergens as well as minor allergens in the extract, which varies with the composition of the biological source material.

The amount of allergen extract in grams to be used for obtaining a desired bio-potency varies with the type of extract in question, and for a given type of extract the amount of allergen extract varies from one batch to another with the actual bio-potency of the extract.

For a given batch of extract, the amount of allergen extract in grams to be used for obtaining a desired bio-potency may be determined using the following procedure:
a) The bio-potency of various amounts of a reference extract is determined using one or more immunological *in vivo* tests to establish a relationship between bio-potency and amount of reference extract. Examples of the said immunological *in vivo* tests are Skin Prick Test (SPT), Conjunctival Provocation Test (CPT), Bronchial Challenge with Allergen (BCA) and various clinical trials in which one or more allergy symptoms is monitored, see for example e.g. Haugaard et al., J Allergy Clin Immunol, Vol. 91, No. 3, pp 709-722, March 1993.
b) On the basis of the established relationship between bio-potency and reference extract, the bio-potency of one or more relevant doses for use in the dosage forms of the invention is selected with due consideration to a balance of the factors of i) the effect of treating or alleviating symptoms of allergy, ii) side effects recorded in the immunological *in vivo* tests, and iii) the variability of i) and ii) from one individual to another. The balancing is done to obtain a maximal adequate therapeutic effect without experiencing an unacceptable level of side effect. The way of balancing the factors are well known to those skilled in the art
   The bio-potency of the one or more relevant doses found may be expressed in any biopotency unit available, such as SQ units, BAU, IR units, IU, cf. above.
c) From the reference extract one or more bio-potency reference standard extracts is prepared and, if used, the bio-potency unit values of the reference standard extracts are calculated on the basis of the bio-potency unit value allocated to the one or more relevant doses, e.g. such a standard for BAU can be obtained from FDA as illustrated below.
d) For the reference standard extracts of each extract type, a number of parameters for evaluating the bio-potency of extracts are selected. Examples of such evaluation parameters are total allergenic activity, the amount of defined major allergens and overall molecular composition of the extract. The total allergenic activity may be measured using an in vitro competitive immunoassay, such as ELISA and MagicLite® luminescence immunoassay (LIA), using a standardised antibody mixture raised against the extract obtained using standard methods, e.g. antibodies raised in mouse or rabbit, or a pool of allergic patients sera. The content of major allergens may e.g. be quantified by rocket immuno-electrophoresis (RIE) and compared to the reference standards. The overall molecular composition may be examined using e.g. crossed immunoelectrophoresis (CIE) and sodium dodecyl sulphate polyacrylamide gel electrophoresis (SDS-PAGE).
e) For a given batch of extract of unknown bio-potency (test extract), the amount of extract to be used for obtaining a desired bio-potency level (effective dose for use in the solid dosage form according to the present invention) may be determined as follows: For each evaluation parameter selected, the test extract is compared with the reference standard extracts using the relevant measurement methods as described above, and on the basis of the measurement results the amount of extract having the desired bio-potency is calculated.

The optimum pH for different allergens in solution span almost the entire pH range as does their isoelectric point (pI). Mixtures of allergens like extracts equally have optimum pH for solubility and stability determined by factors like the concentration of the individual allergens in the extract. Therefore an individual determination of a feasible range of pH for a formulation according to this invention may be envisaged. The optimum pH for the allergen in question is determined by carrying out accelerated stability studies with formulations with different pH. The design of such studies is known to the person skilled in the art.

Allergen extracts should preferably be adjusted to pH between 3.5-10, more preferably 4-9, most preferably 6-9.

SQ-Unit: The SQ-Unit is determined in accordance with the ALK-Abelló A/S "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. This means that 1,000,000 SQ are contained in from 1 mg extract to 10 mg allergen extract, and that 100,000 SQ are contained in from 0.1 mg extract to 1 mg allergen extract. In a similar manner, any SQ dose may be transformed into an allergen extract dose range. On this basis, the above dose ranges given in SQ may be recalculated into dose ranges in mg or µg allergen extract, wherein for the lower SQ limit of a range, the lower limit of the corresponding allergen extract range is used, and wherein for the upper SQ limit of a range, the upper limit of the corresponding allergen extract range is used. The precise allergen amount can be determined by means of immunoassay i.e. total major allergen content and total allergen activity.

Mass unit standardization consists in the quantification of the major(s) allergen(s) present in an allergenic extract intended for clinical use by an allergen-specific immunoasay, mainly 2-site ELISA based on monoclonal antibodies, which uses as reference a preparation whose allergen content is known in mass units (e.g. µg).

BAU (Biological Allergen Units) is biological potency units as determined according to the requirements of the FDA for allergen product described in "Quantitative determination of relative potency of allergenic extracts" ("Methods of the allergen products testing Laboratory" "ELISA competition assay". Page 15, #49N-0012, FDA, October 1993). A dose of 100,000 SQ-Units containing grass extract equals a content of 2600-4700 BAU according to the method above. Likewise, other extracts can be assessed according to the method above.

### Therapeutic methods of the invention

The invention also relates to a method for inducing tolerance in a subject who is allergic to an allergenic agent, the method comprising repeated administrations of single dosage forms isolated from a standardized allergen extract comprising allergens derived from said allergenic agent, wherein the relative amounts of individual allergens in the single dosage forms are kept substantially constant over time.

An embodiment of this aspect is one, wherein the single dosage forms are from an allergen extract which has been matched with either the subject's allergen sensitisation profile or the allergen sensitisation profile of an allergic population, so that 1) the total amount of allergens which constitute allergens in the subject or constitute major allergens in the allergic population does not exceed the maximum acceptable amount of any one single major allergen of said allergenic agent and/or the concentrations of major and minor allergens in the standardized allergen extract are controlled quantitatively, and 2) that one single dosage form of said allergen extract comprises an immunogenically effective amount of each of said allergens. *I.e*. this embodiment utilises standardized extracts disclosed herein in a rational manner, and this is rendered possible because the medical practioneer has access to knowledge of the particular standardized extract and the most relevant target populations that matches each of the standardized extracts and medicaments prepared therefrom.

In a related aspect, the invention also relates to a method for inducing tolerance in a subject who is allergic to an allergenic agent, the method comprising obtaining the allergen sensitisation profile of said subject or of the allergenic population to which the subject belongs, selecting a standardized allergen extract or allergen composition which matches the allergens to which the subject is allergic or which matches the major allergens reactive in the allergenic population, and subsequently administering repeated single dosage forms isolated from the standardized allergen extract or allergen composition to induce tolerance to the allergenic agent, wherein the standardized allergen extract and allergen composition are ones wherein 1) one single dosage form thereof comprises a total amount of major allergens found in said population not exceeding the maximum acceptable amount of any one single major allergen of said allergenic agent and/or the concentrations of major and minor allergens in the standardized allergen extract and allergen composition are controlled quantitatively, and 2) that one single dosage form thereof comprises an immunogenically effective amount of each of said major and optionally minor allergens.

It will be understood, that standardized allergen extracts used in therapy can be broadly applicable (because all allergic subjects react to the same major allergens and that the extract has a controlled composition with respect to major as well as minor allergens). However, in cases where it according to the invention is appropriate to induce tolerance in different populations with different extracts, the therapeutic methods of the invention will entail that the standardized allergen extract is part of a set of at least 2 distinct allergen extracts wherein each distinct extract matches a population according to major allergens reactive in said population. The number of distinct standardized extract can be considerably higher than 2, such as at least 3, at least 4, at least 5, at least 6, at least 7, at least 8, at least 9, or at least 10.

In general, the therapeutic methods of the invention are, with respect to other variables, performed according to methods generally accepted in the art. Typically, the patients will receive the single dosage of the allergen extract in such a way that the dosage is increased over time to reach a maintenance dose, which then normally is administered for the rest of the treatment. Typically, the major allergen maintenance dose for efficacy in subcutaneous immunotherapy is in the range 5-20 µg per injection, whereas the initiation dose is about 10-fold lower than the maintenance dose (10).

So, the vaccines are conventionally administered parenterally, by injection, for example, either subcutaneously, intracutaneously, intradermally, subdermally or intramuscularly. Additional formulations which are suitable for other modes of administration include, oromucosal i.e. oral, buccal, sublinqual, gastrointestinal formulations, suppositories and, in some cases intraperitoneal, intravaginal, anal, epidural, spinal, and intracranial formulations.

The vaccines are administered in a manner compatible with the dosage formulation, and in such amount as will be therapeutically effective and immunogenic. The quantity to be administered depends on the subject to be treated, including, *e.g*., the capacity of the individual's immune system to mount an immune response. Suitable dosage ranges are of the order of several hundred micrograms active ingredient per vaccination with a preferred range from about 0.1 µg to 2,000 µg (even though higher amounts in the 1-10 mg range are contemplated), such as in the range from about 0.5 µg to 2,000 µg or 0.5 µg to 1,000 µg, preferably in the range from 1 µg to 500 µg and especially in the range from about 2.5 µg to 100 µg and 2.5 - 75 µg, with especially preferred range between 10 and 20 µg. Suitable regimens are typified by an initial administration followed by subsequent inoculations or other administrations, typically with increasing dosages of the allergen until a plateau level is reached whereafter the immunizations are maintained at the plateau level. Some oral administrations entail daily immunizations.

The manner of application may be varied widely. Any of the conventional methods for administration of a vaccine are applicable. These include oromucosal application on a solid physiologically acceptable base or in a physiologically acceptable dispersion, parenterally, by injection or the like. The dosage of the vaccine will depend on the route of administration and will vary according to the age of the person to be vaccinated and the formulation of the antigen. As mentioned above, a typical anti-allergy treatment regiment entails early immunizations using small amounts of allergens per administration followed by an increase in allergen dose over time. Another entails oromucosal administration of solid dosage forms containing a mono-dose, preferably without any updosing with different allergen doses. A third possibility is the oromucosal or sublingual administration of a liquid mono-dose, preferably without any up-dosing with different allergen doses.

### Kits of the invention

It will be understood that the practice of the present invention always rely on the interplay between the allergens present in an allergen vaccine and the population/individual to be immunized. In this context, a very important product which is part of the present invention is an anti-allergy kit, said kit comprising
- diagnostic means for profiling an allergic subject so as to determine which allergens derived from an allergenic agent are allergenic in the subject,
- a set of distinct standardized allergen extracts, wherein each of said distinct standardized allergen extracts comprises allergens derived from said allergenic agent, said distinct standardized allergen extracts being defined relative to allergic populations so that 1) each standardized allergen extract comprises major allergens reactive in each such population so that the weight ratio between the most abundant major allergen and any other major allergen reactive in the population does not exceed 30:1 and ii) the ratio between any minor allergen and the most abundant major allergen does not exceed 1:50 or the concentrations of minor allergens are within defined limits.

The diagnostic means may be any suitable diagnostic means known in the art, but will typically be means for performing an immunoassay which is capable of detecting IgE antibodies in a subject which react to a number of different allergens. A preferred immunoassay is an ELISA, e.g. a sandwich ELISA comprising the allergen coated to a solid support, whereafter binding of IgE to the coated allergen is detected by means of a labelled anti-IgE antibody. Any of the types of assays for profiling of allergic subjects described herein can be used, however.

Likewise, the invention also contemplates an anti-allergy kit, said kit comprising
- diagnostic means for profiling an allergic subject so as to determine which allergens derived from an allergenic agent are allergenic in the subject,
- a set of distinct allergen compositions, wherein each of said distinct allergen compositions comprises allergens derived from said allergenic agent, said distinct allergen composition being defined relative to allergic populations so that each allergen composition comprises major allergens reactive in each such population so that i) the weight ratio between the most abundant major allergen and any other major allergen reactive in the population does not exceed 30:1 and ii) the ratio between any minor allergen and the most abundant major allergen does not exceed 1:50 or the concentrations of minor allergens are within defined limits. In this embodiment, the allergens are either isolated allergens or allergens that are recombinantly or synthetically produced.

In a preferred embodiment, the kits of the invention comprise all major allergens of said allergenic agent in the distinct allergen compositions or standardized extracts.

The standardized allergen compositions and extracts of the kits in general share all features already described herein for the standardized allergen extracts and compositions, meaning that all features described herein for the standardized allergen extracts and composition apply mutatis mutandis to the allergen compositions and extracts of the kits.

The invention will be illustrated by means of the following non-limiting examples.

### EXAMPLES

A more complete understanding of the invention can be obtained by reference to the following specific Examples. These Examples are described solely for purposes of illustration and are not intended to limit the scope of the invention. Although specific terms have been employed herein, such terms are intended in a descriptive sense and not for purposes of limitations.

### EXAMPLE 1

This example describes the different patterns of sensitization to two allergens from olive tree pollen (the major allergen Ole e 1 and the minor allergen Ole e 9) shown by patients living in areas with high or low level of exposure to airborne olive tree pollen and the variability of olive tree pollen batches regarding the concentration of these allergens, including the description of a new method to measure the concentration of Ole e 9 in olive tree pollen extracts.

Olive tree pollen is one of the most important causes of respiratory allergies in the Mediterranean region and some areas of North America (12, 13). Ten different allergens have been identified in olive tree pollen up to date (14, 15). All the authors agree on the identification of Ole e 1 as the most important allergen (14, 16, 17, 18). However, for the rest of allergens, prevalence data are controversial, since a number of factors, such as population selected, analytical methods and reagents used to perform the tests, can influence the results obtained (14). In particular, those tests carried out with pollen extracts might lead to incorrect results since, due to the variability of olive tree pollen (14, 19), the concentration of some allergens could be under the detection limit of the assays.

To overcome this problem, the present inventors analyzed the frequencies of sensitization and serum specific-IgE levels using purified proteins at known concentrations in an ADVIA-Centaur equipment, as described below. The proteins tested were Ole e 1 and Ole e 9. Ole e 1 is a 145 amino acid-residue protein (18-20 kDa) that consists of glycosylated and non-glycosylated forms (20). The allergen belongs to a family of proteins, which has been suggested to be related to pollen germination, tube growth and/or pollen germination (21). Ole e 9 consists of a single glycosylated polypeptide chain (434 amino acid residues, 46 kDa) with 1,3-beta-glucanase activity (22), which belongs to the group 2 pathogenesis-related protein family. Ole e 9 consists of two well-defined domains: an N-terminal portion (around 340 residues), containing the catalytic site, and a C-terminal domain (around 100 residues), the function of which is so far unknown (23, 24). Both Ole e 9 domains have been produced, separately, as recombinant proteins in the yeast Pichia pastoris. These recombinant proteins were correctly folded and displayed immunochemical properties similar to their natural counterparts (23,24).

For this study, the present inventors compared the results obtained with sera from patients living in areas (Madrid and Jaén) where similar frequencies of sensitization to olive tree pollen have been reported (25), but with very different levels of atmospheric pollen exposure. Thus, for instance, the maximum daily peak during the olive tree pollen season of year 2004 in Madrid was 70 grains/m³, whereas the value in Jaén was 3354 grains/m³ (data from the web page of the Spanish Society of Allergy and Clinical Immunology: http://www.seaic.es). Moreover, the present inventors have analyzed the content of the allergens Ole e 1 and Ole e 9 in pollen extracts using specific methods based on monoclonal antibodies (mAbs). The method used for measuring Ole e 1 is an adaptation to an ELISA (enzyme-linked immunosorbent assay) format of the original radioimmunoassay method (18). For Ole e 9 determination, new anti-Ole e 9 antibodies have been produced and an ELISA method has been developed. This ELISA uses one anti-Ole e 9 monoclonal antibody adsorbed to the solid phase and anti-Ole e 9 rabbit polyclonal serum as the second antibody, as described below.

In the following paragraphs, a description of the methods used is given and, then, the most relevant results obtained are presented.

### Methods

### Production of anti-Ole e 9 monoclonal antibodies

Ole e 9 allergen, purified from olive tree pollen as previously published (22), was kindly provided by Dr. Rodríguez (Universidad Complutense, Madrid, Spain). Female BALB/c mice (CRIFFA, Barcelona, Spain) were injected intraperitoneally with 16 µg of this allergen in Freund's complete adjuvant. On day 15 and day 30 after the injection, mice were boosted with an identical amount of antigen in Freund's incomplete adjuvant. On day 42, mice were boosted intravenously with the same dose of antigen in PBS and, three days later, spleen cells from immunized mice were fused with P3.X63.Ag8.653 myeloma cells according to the method of Galfré and Milstein (26). Ten days after the fusion, cell culture supernatants were screened for anti-Ole e 9 specific antibodies by an ELISA with the allergen on the solid phase. The P3.X63.Ag8 mouse myeloma culture supernatant was used as a negative control. The positive hybridomas were cloned and subcloned by limiting dilution. Selected monoclonal antibodies were purified from hybridoma culture supernatants using a Protein G Sepharose affinity column (Amersham Biosciences, Uppsala, Sweden), following the manufacturer's instructions. The isotype of the antibodies was determined by ELISA with anti-mouse subclass antisera (Nordic, Tilburg, The Netherlands).

Both monoclonal and polyclonal antibody production (described below) were carried out in accordance with the OECD principles of good laboratory practice (as revised in 1997) and European Council Directive No. 609 (1986).

### Preparation of olive tree pollen extracts

For this example, different batches of olive tree pollen from several suppliers were extracted at a 1:10 (w/v) ratio in phosphate buffer, pH 6.5, with magnetic stirring, at 4°C. The soluble fraction was separated by centrifugation at 22,000 g for 20 min at 4 °C and filtered through 0.22 µm filters (Sartorius AG, Göttingen, Germany).

### Purification of Ole e 9 by affinity chromatography .

An immunosorbent column was prepared by coupling anti-Ole e 9 mAb 1.18 to CNBr-activated Sepharose 4B gel (Amersham Biosciences), following the manufacturer's recommendations. Olive tree pollen extract was passed through the column and, after extensive washing with phosphate buffered saline (PBS), Ole e 9 was eluted using 100 mM glycine, pH 2.5. 1-ml fractions were collected in tubes containing 50 µl of neutralizing buffer (1M Tris buffer, pH 9.0). Fractions were pooled on the basis of protein content, dialyzed against distilled water and stored in aliquot samples at -40°C until use. The purity of affinity-purified Ole e 9 was assessed by SDS-PAGE, and the IgE binding capacity by SDS-PAGE/Immunoblot with a pool of sera from olive tree-allergic patients. Amino acid analysis was carried out using reagents and equipment from Biochrom Ltd. (Cambridge, UK).

### Polyclonal antiserum against Ole e 9

Polyclonal antibodies against Ole e 9 were produced in New Zealand rabbits. Each rabbit was injected intramuscularly with 150 µg of affinit-purified Ole e 9 dissolved in 500 µl of PBS and mixed with the same volume of Freund's adjuvant (complete for the first injection and incomplete for the rest). Injections were repeated every 15 days and sera were collected 10 days after each injection. A rabbit serum pool from two animals was made up of three successive bleedings.

### Ole e 9-ELISA

ELISA plates (Costar, reference 3590, Cambridge, MA, USA) were coated overnight at 4°C with 100 µl of anti-Ole e 9 mAb 18.1 at 10 µg/ml in PBS. After blocking with 1% (w/v) BSA, 0.05% (v/v) Tween 20 in PBS (PBS-BSA-Tween), wells were sequentially incubated with samples and references, anti-Ole e 9 rabbit serum (1/1000 dilution), and goat anti-rabbit immunoglobulin antibodies conjugated with horseradish peroxidase (1/10000 dilution; Calbiochem, San Diego, CA, USA). Samples, controls and reagents were diluted in PBS-BSA-Tween, and all incubations were carried out for 1 h at room temperature with intermediate washes with 0.05% (v/v) Tween 20 in PBS between successive steps. Detection was accomplished by incubation in the dark for 30 minutes with peroxidase substrate buffer (0.012% H₂O₂, 0.66 mg/ml o-phenylenediamine, OPD; DAKO, Glostrup, Denmark). The colour reaction was then stopped by adding 100 µl of 2N HCl and the optical density was read at 492 nm with a 650-nm reference filter. Assays were performed in duplicate. Blocking buffer was used as a negative control. The Ole e 9 content of the samples was obtained by interpolating from a standard curve constructed with eight serial three-fold dilutions of affinity-purified Ole e 9, starting from 15.7 µg/ml.

### Ole e 1-ELISA

Quantification of Ole e 1 was achieved by a 2-site solid-phase ELISA based on Ole e 1-specific mAb (18). ELISA plates (Costar, reference 3590) were coated overnight at 4°C with 100 µl of anti-Ole e 1 mAb OL7 at 5 µg/ml in PBS. After blocking with 1% BSA in PBS for 30 minutes at room temperature, wells were sequentially incubated with samples and reference, biotin-labelled anti-Ole e 1 mAb OL2 (1/1000 dilution) and streptavidin-peroxidase (1/1000 dilution, Amersham Biosciences). All incubations were carried out for 1 h at room temperature with intermediate washes with 0.1% Tween-20 in PBS between successive steps. Finally, the wells were incubated in the dark at room temperature with peroxidase substrate buffer as for Ole e 9-ELISA. The reaction was stopped after 30 minutes with 50 µL of 2M H2SO4, and the optical density per well was measured at 492 nm with a 650 nm reference filter. Assays were performed in duplicate. Blocking buffer was used as a negative control. The Ole e 1 content of the samples was obtained by interpolating from a standard curve (range 0.435 µg/ml to 0.006 µg/ml) constructed with serial 2-fold dilutions of a reference with known Ole e 1 concentration (18).

### SDS-PAGE and IgE-immunoblotting

SDS-PAGE was carried out using non-reducing conditions on a 10-20% tricine-polyacrylamide running gel (Novex, San Diego, CA, USA). The proteins separated by SDS-PAGE were transferred onto nitrocellulose membranes (0.4x7 cm) as described by Towbin et al.(27). After blocking, immunodetection of IgE-binding proteins was achieved by incubating with a 1/3 dilution of patient's serum, followed by sequential incubations with a 1/3000 dilution of mouse anti-human IgE mAb HE-2 ascitic fluid (28) and then with rabbit anti-mouse immunoglobulin antibodies conjugated with horseradish peroxidase (1/5000 dilution, DAKO). IgE-binding proteins were detected by enhanced chemiluminescence following the manufacturer's instructions (ECL, Amersham Biosciences). As negative control, a blot with transferred proteins was incubated with dilution buffer instead of patient's serum.

### RAST-inhibition

Allergenic activity of olive tree pollen extracts was determined by RAST-inhibition. Paper disks were activated with CNBr and sensitized with an olive tree pollen in-house reference extract (batch T272) as indicated (6). A pool of human sera from 20 patients that contained specific IgE to olive tree pollen extract was used throughout the experiments. The patients had a positive skin prick test and clinical history of hypersensitivity to olive tree pollen. A volume of 50 µl of a 1/3 dilution of the serum pool was added per well to a 96-well microtiter plate (Costar, reference 3370) and incubated together with the same volume of 3-fold serial dilutions of samples (olive tree pollen extracts) and in-house reference T272 for 30 minutes at 37°C. One allergen disk per well was then added and incubated for another 3-4 hr at room temperature. After washing the disks 3-times with 0.1% Tween-20 in PBS, about 125,000 cpm/well of ¹²⁵I-labelled anti-human IgE mAb HE-2 (28) was added and incubated overnight at room temperature. Finally, the disks were washed and the bound radioactivity was measured in a gamma counter. The allergenic activity of olive tree pollen extracts was expressed in BU/ml by comparison with the in-house reference T272, which previously had been calibrated in BU/ml by skin prick test (29).

### Biotinylation of allergens

The allergens biotinylated for this example were natural Ole e 1 and the recombinant C- and N-terminal domains of Ole e 9 expressed in *Pichia pastoris.* These allergens had been purified as previously described (17, 23, 24) and kindly provided by Dr. R. Rodríguez (Universidad Complutense, Madrid, Spain). Allergens (250 µg) were dissolved in 0.1M NaHCO₃, pH 8.5 (500 µl) and 5 µl of a solution of Biotin EZ-Link NHS-LC-LC (Pierce, Rockford, IL, USA) at 5 mg/ml in N,N-dimethylformamid was added and let to react for 2 h at 4°C. Afterwards, the excess reagent was separated from the biotinylated allergen by passing the reaction mixture through a NAP5 column (Amersham Biosciences) previously equilibrated with PBS. Elution was accomplished with PBS. The 1-ml fraction containing the biotinylated allergen was diluted with the same volume of glycerol and stored in aliquots at -20°C.

### Specific IgE determination

Allergen-specific IgE in human sera was determined by the ADVIA Centaur-specific IgE assay. Serum samples were from allergic patients living in Madrid (n=49) or Jaén (n=40) with hypersensitivity to olive tree pollen, as demonstrated by clinical history and positive skin prick test to a commercial in vivo diagnostic product (ALK-Abelló, S.A.). Biotin-labelled allergens were obtained as indicated above. For Ole e 9-specific IgE determinations, equimolar amounts of the recombinant C- and N-terminal domains of Ole e 9 were mixed. The optimal dose of biotinylated allergen for the ADVIA Centaur assay was determined by titration of the labelled preparation. An optimal dose of 20 ng per test was found. Calibrators, controls and universal reagent packs (URP) were obtained from ALK-Abelló (Stenloese, Denmark). The instrument and other reagents and consumables were obtained from Bayer Diagnostics (Tarrytown, NY, USA).

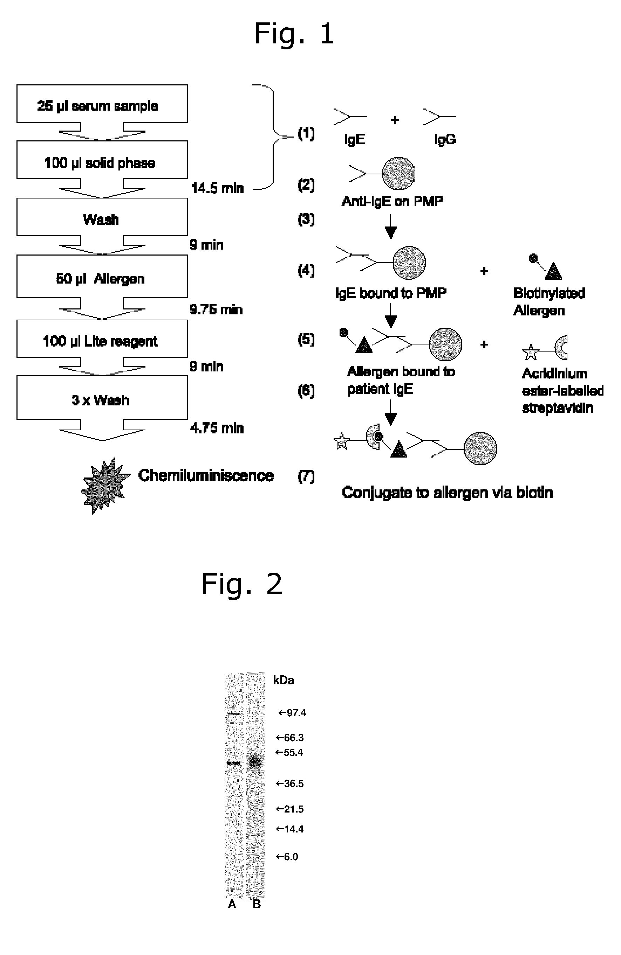

The ADVIA Centaur-specific IgE assay is a reverse sandwich immunoassay using direct chemiluminiscent technology based on the ADVIA Centaur platform from Bayer Diagnostics, which is a continuous and fully automated system where the hands-on labor is restricted to loading URPs containing paramagnetic particles and lite reagent, wash buffer, acid, base, cuvettes, pipette tips, biotynylated allergens and calibrators and bar-coded controls and serum samples. Fig. 1 shows the architecture of the assay (30). The serum sample (25 µl) is dispensed into a cuvette (step 1) and then IgE is allowed to bind to anti-IgE coupled to paramagnetic particles (solid phase) (step 2). After magnetic separation, unbound serum material is washed away (step 3) before biotin-labelled allergen is allowed to bind to patient-specific IgE (step 4). Lite reagent containing acridinium ester-labelled streptavidin binds to bound allergen (step 5), and after washing (step 6) and activation the chemiluminiscent flash emitted from the resulting complexes is measured in a luminometer (step 7). A linear relationship exists between the amount of allergen-specific IgE in the serum sample and the amount of relative light units detected by the system.

The calibration system used in the ADVIA Centaur specific IgE assay is based on a linear algorithm as indicated (30). It has been supposed a slope calibration factor for each allergen equal to 1.

### Results

### Production of anti-Ole e 9 monoclonal antibodies and purification of Ole e 9 by affinity chromatography

Forty monoclonal antibodies of IgG class, specific for Ole e 9, were obtained from the fusion of spleen cells of mice immunized with Ole e 9 and P3.X63.Ag8.653 myeloma cells. One of these mAbs (18.1) was selected to be used for immunoaffinity chromatography and to develop an ELISA to quantitate Ole e 9. Affinity chromatography enabled the present inventors to purify Ole e 9 from an olive tree pollen extract. This method, simpler than other previously described, rendered Ole e 9 at a high purity level. The electrophoretic pattern of immunoaffinity-purified Ole e 9 (Fig. 2A) displayed two bands at 46 and 92 kDa, which correspond to the monomer and dimer forms of the protein, respectively (22). Both bands were immunostained with the sera from olive tree-allergic patients in Western blot (Fig. 2B), showing that the allergen maintained its IgE-binding ability after the purification process. This affinity-purified Ole e 9 was used to produce a polyclonal antiserum in rabbit.

### ELISA for Ole e 9 quantitation

The method developed by the present inventors to quantify Ole e 9 consists in a sandwich ELISA with the mAb 18.1 on the solid phase and an anti-Ole e 9 rabbit serum as the second antibody. Detection of the rabbit antibodies bound is accomplished with goat anti-rabbit immunoglobulin antibodies conjugated with horseradish peroxidase.

The specificity of the method was evaluated against a large battery of extracts from pollens of grasses, trees, and weeds. Only the extracts from pollens belonging to the Oleaceae family (Fraxinus excelsior, Syringa vulgaris and Ligustrum vulgare) gave a significant response in the ELISA method, but the rest were negative in the assay (data not shown). These results evidence that proteins homologues to Ole e 9 are present in other Oleaceae plants.

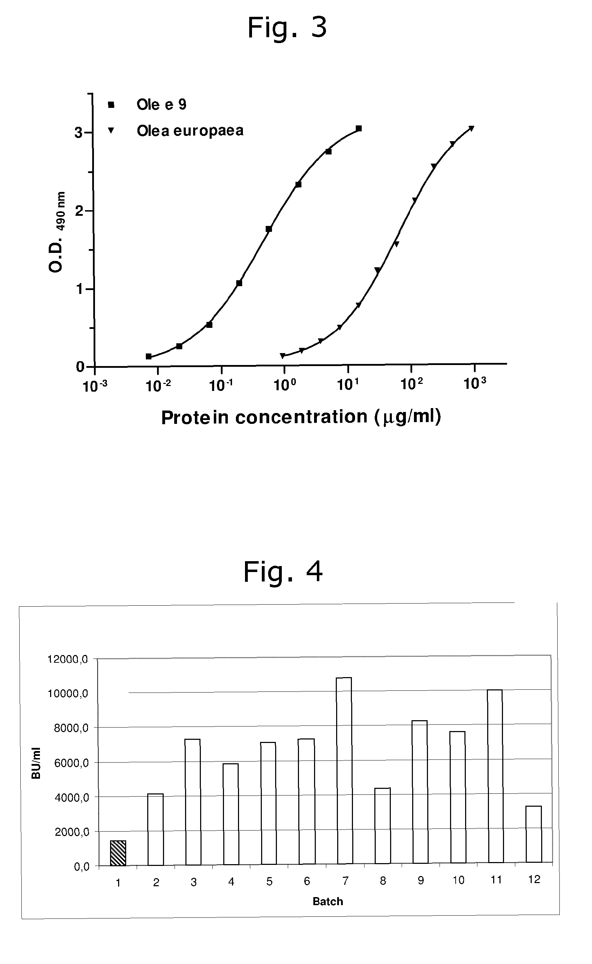

The dose-response curves obtained in the ELISA with affinity-purified Ole e 9 and olive tree pollen extract were parallel (Fig. 3), showing that the allergen had not been altered during the purification process and that the assay was measuring the same molecule in the different preparations. The concentration of affinity-purified Ole e 9 was accurately measured by quantitative amino acid analysis, and this preparation was used as the primary standard in the ELISA method.

### Variability of olive tree pollen

The variability of olive tree pollen was studied in twelve different batches using the ELISA described above for Ole e 9 quantitation, an ELISA for Ole e 1 quantitation and RAST-inhibition for allergenic activity. Results obtained are gathered in Table 1.

**Table 1. Values of allergenic activity (in BU/ml), and Ole e 1 and Ole e 9 content (in µg/ml) in batches of olive tree pollen. Samples were analyzed as described in Methods.**

| **Sample** | **Supplier** | **Batch** | **BU/ml** | **µg Ole e 1/ml** | **µg Ole e 9/ml** |
|---|---|---|---|---|---|
| **1** | Biopol | 02-1465 | 1467 | 469 | 754 |
| **2** | Biopol | 02-1381 | 4138 | 843 | 346 |
| **3** | Crystal | 198Y4H01 | 7296 | 3599 | 1449 |
| **4** | Biopol | 02-1379 | 5837 | 1636 | 531 |
| **5** | Biopol | 02-1377 | 7086 | 1879 | 495 |
| **6** | Allergón | 325010802 | 7260 | 5429 | 191 |
| **7** | Biopol | 02-1382 | 10781 | 7538 | 256 |
| **8** | Biopol | 02-1378 | 4356 | 7293 | 162 |
| **9** | Biopol | 01-1841 | 8276 | 11287 | 141 |
| **10** | Mediterráneo | F1 | 7623 | 7637 | 68 |
| **11** | Greer | 23ii123SD | 10019 | 11569 | 79 |
| **12** | Biopol | 02-1380 | 3267 | 3668 | 9 |

A high variability was observed in the allergenic activity. The activity of the most potent extract was almost ten times the activity of the extract with the lowest activity. This one (batch sample 1) should be rejected for production of an olive tree allergy vaccine according to the specifications of the present assignee's Production Department because of its low activity (Fig. 4).

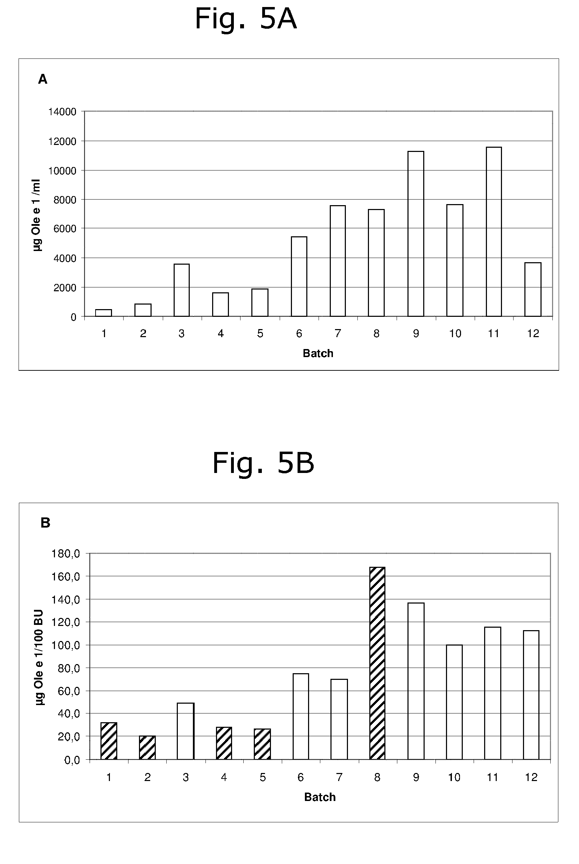

A higher variability was observed in Ole e 1 content (Fig. 5A). Thus, a ratio of 25 was estimated between the sample with the highest Ole e 1 content (batch sample 11) and the one with the lowest content (batch sample 1). As a concentration of 60 µg of Ole e 1/ml has been established for an activity of 100 BU/ml, with an acceptance range between 30 and 120 µg/ml (9), four additional batches (samples 2, 4, 5, and 8) were found to be out of specifications (Fig. 5B).

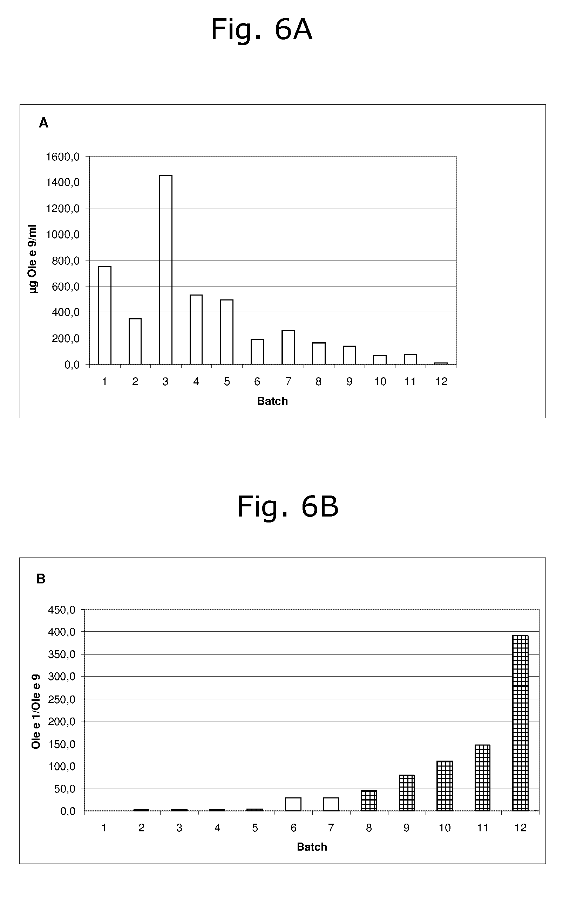

Moreover, and unexpectedly, extremely high variability was found when the samples were analyzed for Ole e 9 content. Concentration of Ole e 9 in sample 3 was 161-fold higher than the concentration in batch sample 12 (Fig. 6A). It should be noted that batches with differences in Ole e 9 content as high as 161-times could be released according to current standardization methods, since both batches where within specifications regarding the former criteria.

When the ratio between the concentration of both allergens was estimated, an even higher variability was observed (Fig. 6B). Moreover, a significant inverse correlation between the Ole e 1 and Ole e 9 contents in olive tree pollen batches was observed (rₛ = -0.7203, p = 0.0082). The Ole e 1/Ole e 9 ratio ranged from 0.6 to 390.4, with a geometric mean value of 17.5. If an acceptance criteria were established based on this value, with an accepted range between 8.75 and 35.0, five additional batches should be rejected (samples 3, 9, 10, 11, and 12). Therefore, only two batches (samples 6 and 7) would fulfil the acceptance criteria.

In conclusion, the new method developed enabled the present inventors to detect an unknown source of variability in batches of olive tree pollen. This high variability in Ole e 9 content, previously unnoticed, might have been the origin of severe adverse reactions to olive tree immunotherapy treatments.

### Pattern of sensitization to Ole e 1/Ole e 9 in regions with different exposure levels to olive tree pollen

The sensitization of patients living in areas with similar prevalence of allergy to olive tree but with very different level of intensity of exposure, i.e. Madrid and Jaén, was studied by measuring the levels of specific IgE to allergens Ole e 1 and Ole e 9, as described in Methods. The results obtained for the prevalence of sensitization and the median values of specific IgE levels found in patients from each area are shown in Table 2.

**Table 2. Prevalence of sensitization and median values of specific IgE levels to allergens Ole e 1 and Ole e 9 in olive tree-allergic patients from Madrid and Jaén. Patients with specific IgE level >0.35 KU/L were considered positive. Median values are estimated considering only the positive patients.**

| | | **Ole e 1** | | **Olee9** | |
|---|---|---|---|---|---|
| **Area** | **N** | **Prevalence (%)** | **Median IgE (KU/L)** | **Prevalence (%)** | **Median IgE (KU/L)** |
| **Madrid** | 35 | 60.0 | 5.9 | 5.7 | 13.0 |
| **Jaén** | 40 | 80.0 | 10.2 | 35.0 | 10.0 |

This data clearly evidences that Ole e 9 is not a prevalent allergen in Madrid, whereas it is far more prevalent in Jaén. Therefore, it can be concluded that patients tend to produce IgE antibodies to Ole e 9 when the intensity of exposure increases. Moreover, the specific IgE levels to this allergen in the sensitized patients from both Jaén and Madrid are similar to those found for Ole e 1, showing that Ole e 9 has a great clinical relevance for those patients. In addition, the present inventors did not find any patients sensitized to Ole e 9 without simultaneous sensitization to Ole e 9. This suggests that Ole e 9 may be considered as a marker of clinical severity of allergy to olive tree pollen. Obviously, patients with this pattern of allergen recognition are in a high risk of suffering from adverse reactions if an allergy vaccine with a high content of Ole e 9 is administered to them. Because of the extreme variability in Ole e 9 content of olive tree extracts shown above and the high specific IgE levels found in some patients, this allergen has probably been the agent responsible for clustered adverse reactions occasionally reported in Jaén.

In conclusion, the present inventors have demonstrated that allergens considered as minor may have a very relevant clinical significance for certain populations of patients. To minimize the risk of eliciting adverse reactions when these patients are subjected to an immunotherapy treatment, the present inventors propose the implementation of methods to measure the concentration of minor allergens with, in combination with methods for major allergen quantitation and total allergenic activity, will allow a proper selection of starting materials and the production of extracts with allergen ratios adequate to the patient sensitization profile. These extracts with controlled concentration of minor allergens will increase the safety of allergen vaccines and, probably, also their efficacy.

### EXAMPLE 2

This example describes the variability of the concentration of the allergen Phl p 6 in extracts from Phleum pratense pollen and the close immunochemical relationship of this allergen with the cross-reactive major allergen Phl p 5. A new ELISA method to measure the concentration of Phl p 6 in pollen extracts that may be applied for the control of this allergen in allergy vaccines is presented.

Grass pollens are one of the most important airborne allergen sources worldwide. Allergic sensitization to grass pollen may affect as many as 20% of the general population and up to 40% of atopic individuals (31). To date, eleven different groups of grass pollen allergens have been identified and characterized from one or more species. Group 5 grass pollen allergens have been identified in many members of the Pooideae subfamily and, together with Group 1 allergens, they are the most prominent allergens of grass pollen (31). Group 6 grass pollen allergens have been identified only in Phleum pratense and Poa pratensis up to date. Phl p 6 from P.pratense is an acidic, non-glycosylated 13-kDa protein that was first observed as a protein copurifying with and cross-reactive to Phl p 5, and was referred to as Ag19 (32). Sequence comparison of Phl p 6 and Phl p 5 allergens showed a high degree of homology in the N- and C-terminal regions (33), and immunoabsorption experiments with sera from grass allergic patients demonstrated that Phl p 5 and Phl p 6 share one or more IgE-binding epitopes (34).

Regarding sensitization prevalence to Phl p 6, very dissimilar values have been reported in the literature. Thus, Vrtala et al. found that serum IgE from 75% of grass pollen-allergic patients reacted with the recombinant allergen expressed in E. coli (35). In contrast, Westritschnig *et al.* reported a 15% prevalence in a study carried out in Zimbabwe (36). Intermediate values have been reported for two studies carried out in Italy: 68% in a study with a cohort of 77 patients (37), and 44% in a study with 749 patients (38). Similarly, Ghunaim et al. have studied the prevalence of sensitization in 58 grass pollen allergic patients classified into three groups according to their IgE antibody profile: a grass pollen group only (27% sensitization to recombinant Phl p 6), a grass and tree pollen group (24%), and a grass, tree and compositae pollen group (57%) (39). In addition to the higher value in prevalence, the median of the values of specific IgE to Phl p 6 were higher in the last group. Interestingly, in a recent publication, the IgE reactivity to individual allergens in sera from subjects sensitized to timothy living in Finnish and Russian Karelia has been studied, and an extremely significant difference in prevalence of sensitization to rPhl p 6 has been disclosed (47% in Finnish Karelia vs. 0% in Russian Karelia) (40). As in the case of Ole e 9, these differences in prevalence of sensitization to Phl p 6 might be reflecting differences in exposure levels, polysensitization, or other factors, that cause a particular higher response to this allergen in certain populations. Consequently, and taking into account the considerations above regarding the presence of common IgE-binding epitopes in Phl p 6 and the major allergen Phl p 5, vaccines to be administered to these populations of patients should be controlled in terms of concentration of Phl p 6.

In this example, the present inventors describe the production of monoclonal antibodies against natural and recombinant Phl p 6. The cross-reactivity of these mAbs against rPhl p 5 was evaluated, and one mAb, which recognizes Phl p 6 but not Phl p 5, was selected to develop an ELISA method to quantify Phl p 6. Using this method, the variability of Phl p 6 content in P. pratense extract has been evaluated.

In the following paragraphs, a description of the methods used is given and, then, the most relevant results obtained are presented.

### Methods

### Production of anti-Phl p 6 monoclonal antibodies

Production of anti-Phl p 6 monoclonal antibodies was performed essentially following the same procedure as described in Example 1. Immunization of mice was carried out with natural Phl p 6 isolated from pollen and recombinant Phl p 6 expressed in Pichia pastoris (41). Cell culture supernatants were screened for anti-Phl p 6 specific antibodies by an ELISA with the natural or the recombinant allergen on the solid phase. To test the recognition of Phl p 5 by the anti-Phl p 6 mAbs, a similar assay was carried out with Phl p 5 (41) adsorbed to the solid phase. Selected monoclonal antibodies were purified from hybridoma culture supernatants using a Protein G Sepharose affinity column (Amersham Biosciences), following the manufacturer's instructions.

### Polyclonal antiserum against Phleum pratense extract

Polyclonal antibodies against P. pratense pollen extract were produced in New Zealand rabbits. Each rabbit was injected intramuscularly with 2 mg of extract dissolved in 500 µl of PBS and mixed with the same volume of Freund's adjuvant (complete for the first injection and incomplete for the rest). Injections were repeated every 15 days and sera were collected 10 days after each injection. A rabbit serum pool from four animals was made up of three successive bleedings.

### Preparation of P. pratense pollen extracts

Different batches of P. pratense pollen from several suppliers were extracted as described for olive tree pollen extracts in Example 1. Phl p 5 content was determined by ELISA as previously described (42).

### Phl p 6-ELISA

ELISA plates (Costar, reference 3590) were coated overnight at 4°C with 100 µl of anti-Phl p 6 mAb R1.11 at 10 µg/ml in PBS. After blocking with 1% (w/v) BSA, 0.05% (v/v) Tween 20 in PBS (PBS-BSA-Tween), wells were sequentially incubated with samples and references, anti-P. pratense rabbit serum (1/5000 dilution), and goat anti-rabbit immunoglobulin antibodies conjugated with horseradish peroxidase (1/15000 dilution; Calbiochem). Samples, controls and reagents were diluted in PBS-BSA-Tween, and all incubations were carried out for 1 h at room temperature with intermediate washes with 0.05% (v/v) Tween 20 in PBS between successive steps. Detection was accomplished by incubation in the dark for 30 minutes with peroxidase substrate buffer (0.012% H₂O₂, 0.66 mg/ml OPD; DAKO). The color reaction was then stopped by adding 100 µl of 2N HCl and the optical density was read at 492 nm with a 650-nm reference filter. Assays were performed in duplicate. Blocking buffer was used as a negative control. The Phl p 6 content of the samples was obtained by interpolating from a standard curve constructed with ten serial two-fold dilutions of nPhl p 6, starting from 127 ng/ml.

### Results

### Production of anti-Phl p 6 monoclonal antibodies and study of their specificity

Nineteen mAbs against nPhl p 6 and 20 mAbs against rPhl p 6 were obtained from the fusions of P3.X63.Ag8.653 myeloma cells with spleen cells of mice immunized with the natural or the recombinant allergen, respectively. The specificity of these mAbs was tested by ELISA against both forms of Phl p 6 and Phl p 5. All the mAbs equally recognized the recombinant and the natural Phl p 6, corroborating that the recombinant form was correctly folded (Table 3).

**Table 3. Binding of anti-Phl p 6 monoclonal antibodies to Phl p 5.**

| **Solid phase** | **Mabs anti-nPhl p 6** | | | | | | | | | | | | | | | | | | | |
|---|---|---|---|---|---|---|---|---|---|---|---|---|---|---|---|---|---|---|---|---|
| | **N1.1** | **N1.2** | **N1.3** | **N1.4** | **N1.5** | **N1.6** | **N1.7** | **N1.8** | **N1.9** | **N1.10** | **N1.11** | **N1.12** | **N1.13** | **N1.14** | **N1.15** | **N1.16** | **N1.17** | **N1.18** | **N1.19** | |
| **rPhl p 5** | 0.039* | 0.008* | 3.721 | 0.009* | 3.668 | 3.750 | 3.836 | 3.773 | 0.048* | 0.013* | 0.063* | 3.443 | 3.428 | 3.513 | 3.749 | 0.016* | 0.196* | 0.033* | 3.678 | |

| **nPhl p 6** | 3.556 | 3.706 | 3.752 | 3.694 | 3.727 | 3.680 | 3.746 | 3.833 | 3.778 | 3.639 | 3.668 | 3.646 | 3.507 | 3.713 | 3.771 | 3.692 | 3.824 | 3.638 | 3.795 | |
|---|---|---|---|---|---|---|---|---|---|---|---|---|---|---|---|---|---|---|---|---|
| **rPhl p 6** | 3.330 | 3.860 | 3.903 | 3.799 | 3.737 | 3.685 | 3.680 | 3.829 | 3.689 | 3.774 | 3.695 | 3.747 | 3.495 | 3.832 | 3.767 | 3.803 | 3.708 | 3.713 | 3.778 | |
| **Mabs anti-rPhl p 6** | | | | | | | | | | | | | | | | | | | | |
| **Solid phase** | **R1.1** | **R1.2** | **R1.3** | **R1.4** | **R1.5** | **R1.6** | **R1.7** | **R1.8** | **R1.9** | **R1.10** | **R1.11** | **R1.12** | **R1.13** | **R1.14** | **R1.15** | **R1.16** | **R1.17** | **R1.18** | **R1.19** | **R1.20** |
| **rPhl p 5** | 3.699 | 3.553 | 0.014* | 3.541 | 1.060$ | 0.753$ | 3.705 | 3.590 | 0.024* | 3.634 | 0.010* | 3.687 | 3.703 | 2.813 | 0.014* | 0.417$ | 0.012* | 3.547 | 3.704 | 3.600 |
| **nPhl p 6** | 3.813 | 3.773 | 3.804 | 3.717 | 3.742 | 3.689 | 3.811 | 3.807 | 3.891 | 3.760 | 3.791 | 3.838 | 3.687 | 3.749 | 3.746 | 3.687 | 3.834 | 3.542 | 3.769 | 3.866 |
| **rPhl p 6** | 3.682 | 3.759 | 3.625 | 3.741 | 3.761 | 3.520 | 3.652 | 3.690 | 3.642 | 3.632 | 3.818 | 3.728 | 3.687 | 3.767 | 3.677 | 3.691 | 4.000 | 3.610 | 4.000 | 3.687 |

Values of O.D. at 490 nm in a direct ELISA with pure allergens on the solid phase. Wells were sequentially incubated with hybridoma culture supernatants (1:2 dilution), peroxidase-conjugated rabbit anti-mouse IgG antibodies and OPD, as described in Methods. Cells marked with asterisks (*) indicate the mAbs that do not recognize Phl p 5; cells marked with dollar signs ($) show the mAbs that bind Phl p 5 with low affinity.

In the same way, most anti-Phl p 6 mAbs also bound to Phl p 5 with a high affinity, which supports the antigenic similarity between these proteins. Only 14 mAbs out of 39 did not bind to Phl p 5, and three other bound weakly (Table 3). One of the mAbs that recognizes an epitope of Phl p 6 not shared with Phl p 5 (mAb R1.11) was selected to develop an ELISA method to quantitate Phl p 6.

### ELISA for Phl p 6 quantitation

The method developed by the present inventors to quantify Phl p 6 consists in a sandwich ELISA with the mAb R1.11 on the solid phase and an anti-P. pratense rabbit serum as the second antibody. Detection of the rabbit antibodies bound is accomplished with goat anti-rabbit immunoglobulin antibodies conjugated with horseradish peroxidase.

The specificity of the method against Phl p 5 was evaluated and, as expected from the specificity assay of the mAb R1.11, no optical density was observed even at concentrations of Phl p 5 as high as 10 µg/ml (Fig. 7). The dose-response curves obtained in the ELISA with the natural allergen, used as a primary standard, and P. pratense pollen extracts were parallel (Fig. 7), showing that the allergen had not been altered during the purification process and that the assay was measuring the same molecule in the different preparations. Consequently, the method has been proven to be useful for measuring Phl p 6 without interferences from the presence of Phl p 5, in spite of its antigenic similarity.

### Variability of P. pratense pollen

A preliminary study on the variability of P.pratense pollen has been carried out with 6 extracts from different batches of Phleum pratense (Table 4).

**Table 4. Concentration of Group 5 and Group 6 allergens in extracts from P.pratense pollen, determined by ELISA techniques, as described in Methods.**

| Extract | Phl p 5 (µg/ml) | Phl p 6 (µg/ml) | Phl p 6/Phl p 5 |
|---|---|---|---|
| Phl A | 378.0 | 306.4 | 0.81 |
| Phl B | 94.2 | 61.4 | 0.65 |
| Phl C | 970.0 | 641.1 | 0.66 |
| Phl D | 1049.3 | 534.0 | 0.51 |
| Phl E | 1179.0 | 680.0 | 0.58 |
| Phl F | 736.3 | 770.6 | 1.05 |

The variability observed for the concentration of both allergens Group 5 and Group 6 was of the same order of magnitude (about 12.5 times). The variability in the ratio Group 6 to Group 5 ranged from 0.51 to 1.05. Although the number of samples analyzed so far is not enough to draw definitive conclusions, it seems that the variability of Phl p 6 is not as high as the data presented for Ole e 9. Nevertheless, a variation of 12.5 times in concentration might be sufficient to cause adverse reactions if, in addition, the present inventors take into account the fact that IgE-binding epitopes on Phl p 6 are cross-reactive with Phl p 5. Therefore, an additive effect of the variations of concentrations of these two allergens might represent an elevated risk of eliciting adverse reactions to P. pratense allergy vaccines. Consequently, the present inventors propose that the concentration of the minor allergen Phl p 6 should be measured and the ratio Phl p 6/Phl p 5 adjusted within certain limits to minimize the risk of such adverse reactions. The ELISA method presented herein has been shown to be useful to that effect. Thus, it can be used for selection of proper raw materials and for quality controls of allergen products that could be prepared by mixing of different pollen batches to reach the desired composition.

### EXAMPLE 3

This example describes the variability in the individual responses of patients allergic to the mould Alternaria alternata. This example is presented only to show that individual patients may produce a strong IgE-response to minor allergens in a number of different allergenic extracts, apart from those mentioned in the previous examples. Consequently, the concentration of such allergens should be controlled by adequate quantitation methods to minimize the risk of adverse reactions.

Allergenic mould extracts show a high variability as a result of their intrinsic complexity. The type of fungal strain, different culture conditions and different extraction procedures are the most important causes of such heterogeneity. Ten different allergens have been identified so far (43). The only allergen described with a frequency of sensitization higher than 50% is Alt a 1 (43). The present inventors developed a mAb-based ELISA method to quantify this allergen (44). Nevertheless, from the results presented, the present inventors suggest the convenience of controlling minor allergens in these extracts that could be clinically relevant for some patients.

### Methods and results

Alternaria alternata raw materials consist of mycelia and spores obtained from cultivation of several strains, which had previously been selected on the basis of allergenic activity and protein profile.

The Alternaria raw material was extracted as the olive tree pollen materials in Example 1 to perform IgE-immunoblotting experiments. The results are shown in Fig. 8. All the six patients had IgE against Alt a 1(m.w. in non-reduction conditions about 33 kDa). However, two patients (33%) recognized a protein of about 22 kDa (which probably corresponds to Alt a 7), and 3 patients (50%) recognized a protein of m.w. below 20 kDa (probably Alt a 6). It should be noted that the intensity of staining, which is a measure of specific IgE level, is very high in those patients, and comparable to the intensity of the Alt a 1 band. This implies that minor allergens could also be involved in adverse reactions to Alternaria immunotherapy and, in consequence, their concentrations should be controlled in products intended for clinical use.

### LIST OF REFERENCES

1) Jarvis D, Burney P (1997) Epidemiology of atopy and atopic disease. In Allergy and Allergic Diseases, 1st edn, pp 1208-24. Blackwell Science, Oxford.
2) Bousquet J, Lockey RF, Malling H-J. WHO Position Paper. Allergen Immunotherapy: therapeutic vaccines for allergic diseases. Allergy 1998; 53 Suppl: 1-42.
3) Ishizaka K, Ishizaka T, Hornbrook MM. Physicochemical properties of reaginic antibody. V. Correlation of reaginic activity with gamma-E-globulin antibody. J Immunol 1966; 97:840-53.
4) Johansson SG, Bennich H. Immunological studies of an atypical (myeloma) immunoglobulin. Immunology 1967; 13: 381-94.
5) Lowenstein H. Physico-chemical and immunochemical methods for the control of potency and quality of allergenic extracts. Arb Paul Ehrlich Int 1980; 75:122-32.
6) Ceska M, Eriksson R, Varga JM. Radioimmunosorbent assay of allergens. J Allergy Clin Immunol 1972; 49:1-9.
7) Lombardero M, Carreira J, Duffort O. Monoclonal antibody based radioimmunoassay for the quantitation of the main cat allergen (Fel d I or Cat-1). J Immunol Meth 1988; 108:71-6.
8) Calabozo B, Duffort O, Carpizo JA, Barber D, Polo F. Monoclonal antibodies against the major allergen of Plantago lanceolata pollen, Pla I 1. Affinity chromatography purification of the allergen and development of an ELISA method for Pla I 1 measurement Allergy 2001; 56:429-35.
9) Carreira J, Lombardero M, Ventas P. New developments in in vitro methods. Quantification of clinically relevant allergens in mass units. Arb Paul Ehrlich Inst Bundesant Sera Impstoffe Frank AM 1994; 87:155-64.
10) Bousquet J, Lockey RF, Malling HJ. Allergen immunotherapy: therapeutic vaccines for allergic diseases. A WHO Position Paper. J Allergy Clin Immunol 1998; 102:558-62.
11) Lowenstein H. Quantitative immunoelectrophoretic methods as a tool for the identification and analysis of allergens. Progr Allergy 1978; 25:1-62.
12) Macchia L, Caiaffa MF, D'Amato G, Tursi A. Allergenic significance of Oleaceae pollen. In: D'Amato G, Spieksma FTM, Bonini S, editors. Allergenic pollen and pollinosis in Europe. Oxford: Blackwell; 1991. p. 87-93.
13) D'Amato G, Liccardi G. The increasing trend of seasonal respiratory allergy in urban areas. Allergy 2002; 57 Suppl 71:35-6.
14) Rodríguez R, Villalba M., Batanero E, González EM, Monsalve RI, Huecas S, Tejera ML, Ledesma A. Allergenic diversity of the olive tree pollen. Allergy 2002; 57 Suppl 71:6-16.
15) Barral P, Batanero E, Palomares O, Quiralte J, Villalba M, Rodríguez R. A major allergen from pollen defines a novel family of plant proteins and shows intra- and interspecie croos-reactivity. J Immunol 2004; 172:3644-51.
16) Lauzurica P, Maruri N, Galocha B, González J, Díaz R, Palomino P, Hernández D, García R, Lahoz C. Olive tree (Olea europaea)-pollen allergens. II. Isolation and characterization of two major allergens. Mol Immunol 1988; 25: 337-44.
17) Villalba M, López-Otín C, Martín-Orozco E, Monsalve RI, Palomino P, Lahoz C, Rodríguez R. Isolation of three allergenic fractions of the major allergen from Olea europaea pollen and N-terminal amino acid sequence. Biochem Biophys Res Commun 1990; 172:523-8.
18) Lombardero M, Quirce S, Duffort O, Barber D, Carpizo J, Chamorro MJ, Lezaun A, Carreira J. Monoclonal antibodies against Olea europaea major allergen: allergenic activity of affinity-purified allergen and depleted extract and development of a radioimmunoassay for the quantitation of the allergen. J Allergy Clin Immunol 1992; 89:884-94.
19) Barber D, Carpizo J, García-Rumbao MC, Polo F, Juan F. Allergenic variability in Olea pollen. Ann Allergy 1990; 64:43-6.
20) Villalba M, Batanero E, López-Otin C, Sánchez LM, Monsalve RI, González de la Pena MA, Lahoz C, Rodríguez R. The amino acid sequence of Ole e 1, the major allergen from olive tree (Olea europaea) pollen. Eur J Biochem 1993; 216:863-9.
21) Alché JD, Castro AJ, Olmedilla A, Fernández MC, Rodríguez R, Villalba M, Rodríguez-García MI. The major olive pollen allergen (Ole e I) shows both gametophytic and sporophytic expression during anther development, and its synthesis and storage takes place in the RER. J Cell Sci 1999; 112:2501-9.
22) Huecas S, Villalba M, Rodríguez R. Ole e 9, a major olive pollen allergen is a 1,3-beta-glucanase. Isolation, characterization, amino acid sequence, and tissue specificity. J Biol Chem 2001; 276:27959-66.
23) Palomares O, Villalba M, Rodríguez R. The C-terminal segment of the 1,3-beta-glucanase Ole e 9 from olive (Olea europaea) pollen is an independent domain with allergenic activity: expression in Pichia pastoris and characterization. Biochem J 2003; 369:593-601.
24) Palomares O, Villalba M, Batanero E, Barderas R, Barral P, Rodríguez R. Recombinant production and characterization of the N-terminal domain of the major olive allergen Ole e 9 [Abstract book]. Paris, France: XXII EAACI Congress 2003: 384. (abstract # 1358)
25) Alergologica. Factores epidemiológicos, clínicos y socioeconómicos de las enfermedades alérgicas en España. Edited by SEAIC and Alergia e Immunología Abelló S.A. Madrid 1995, pp. 257-79.
26) Galfré G, Milstein C. Preparation of monoclonal antibodies: strategies and procedures. In: Langone JJ, van Vunakis H; editors. Methods in enzymology. vol 73, part B. New York: Academic Press, 1981: 1-46.
27) Towbin H, Staehelin T, Gordon J. Electrophoretic transfer of proteins from polyacrylamide gels to nitrocellulose sheets: procedure and some applications. Proc Natn Acad Sci USA 1979; 76:4350-4.
28) Sánchez-Madrid F, Morago G, Corbí AL, Carreira J. Monoclonal antibodies to three distinct epitopes on human IgE: their use for determination of allergen-specific IgE. J Immunol Methods 1984; 73: 367-78.
29) Lombardero M, González R, Duffort O, Juan F, Ayuso R, Ventas P, Cortés C, Carreira J. Evaluación de la actividad biológica y composición alergénica de extractos alergénicos. Allergol. Immunopathol. 1986;14:189-98.
30) Petersen AB, Gudmann P, Milvang-Gronager P, Morkeberg R, Bogestrand S, Linneberg A, Johansen N. Performance evaluation of a specific IgE assay developed for the ADVIA centaur immunoassay system. Clin Biochem 2004; 37:882-92.
31) Andersson K, Lidholm J. Characteristics and immunobiology of grass pollen allergens. Int Arch Allergy Immunol 2003; 130:87-107.
32) Lowenstein H. Isolation and partial characterization of three allergens of timothy pollen. Allergy 1978; 33:30-41.
33) Petersen A, Bufe A, Schramm G, Schlaak M, Becker WM. Characterization of the allergen Group VI in timothy grass pollen (Phl p 6). II. cDNA cloning of Phl p 6 and structural comparison to grass Group V. Int Arch Allergy Immunol 1995; 108: 55-9.
34) Petersen A, Bufe A, Schlaak M, Becker WM. Characterization of the allergen Group VI in timothy grass pollen (Phl p 6). I. Immunological and biochemical studies. Int Arch Allergy Immunol 1995; 108: 49-54.
35) Vrtala S, Fischer S, Grote M, Vangelista L, Pastore A, Sperr WR, Valent P, Reichelt R, Kraft D, Valenta R. Molecular, immunological, and structural characterization of Phl p 6, a major allergen and P-particle-associated protein from timothy grass (Phleum pratense) pollen. J Immunol 1999; 163:5489-96.
36) Westritschnig K, Sibanda E, Thomas W, Auer H, Aspock H, Pittner G, Vrtala S, Spitzauer S, Kraft D, Valenta R. Analysis of the sensitization profile towards allergens in central Africa. Clin Exp Allergy 2003; 33:22-7.
37) Rossi RE, Monasterolo G, Monasterolo S. Measurement of IgE antibodies against purified grass-pollen allergens (Phl p 1, 2, 3, 4, 5, 6, 7, 11, and 12) in sera of patients allergic to grass pollen. Allergy 2001; 56:1180-5.
38) Mari A. Skin test with a timothy grass (Phleum pratense) pollen extract vs. IgE to a timothy extract vs. IgE to rPhl p 1, rPhl p 2, nPhl p 4, rPhl p 5, rPhl p 6, rPhl p 7, rPhl p 11, and rPhl p 12. Clin Exp Allergy 2003; 33:43-51.
39) Ghunaim N, Grönlund H, Kronqvist M, Grönneberg R, Söderström L, Ahlstedt S, van Hage-Hamsten M. Antibody profiles and self-reported symptoms to pollen-related food allergens in grass pollen-allergic patients from northern Europe. Allergy 2005; 60:185-91.
40) Moverare R, Petays T, Vartiainen E, Haatela T. IgE reactivity pattern to timothy and birch pollen allergens in Finnish and Russian Karelia. Int Arch Allergy Immunol 2005; 136:33-8.
41) Stovhase RB, Meno K, Olsen M, Friberg L, Lund K, Lenhard T. Biochemical characterization of recombinant Phl p 6 and comparison with natural Phl p 6 and natural Phl p 5 [Abstract]. J Allergy Clin Immunol 2004; 113:S227.
42) Ramírez J, Obispo TM, Duffort O, Carpizo JA, Chamorro MJ, Barber D, Ipsen H, Carreira J, Lombardero M. Group 5 determination in Pooideae grass pollen extracts by monoclonal antibody-based ELISA. Group 5 determination in Pooideae grass pollen extracts by monoclonal antibody-based ELISA. Correlation with biologic activity. Allergy 1997; 52: 806-13.
43) Achatz G, Oberkofler H, Lechenauer E, Simon B, Unger A, Kandler D, Ebner C, Prillinger H, Kraft D, Breitenbach M. Molecular cloning of major and minor allergens of Alternaria alternata and Cladosporium herbarum. Mol Immunol 1995; 32:213-27.
44) Duffort O, Barber D, Polo F. Ensayo de cuantificación del alergeno mayoritario de Alternaria alternata, Alt a 1. Alergol Immunol Clin 2002; 17: 162-8.

### NUMBERED EMBODIMENTS

The invention also relates to the following numbered embodiments:
Embodiment 1. A method for the preparation of a standardized extract of an allergenic agent, comprising
   - obtaining an allergen containing extract derived from said allergenic agent,
   - determining the relative concentrations of major and minor allergens in said allergen containing extract, wherein said major and minor allergens are defined according to the allergen sensitisation profiles obtained from a population allergic to the allergenic agent,
   - adjusting the concentrations of said major and minor allergens to obtain said standardized extract so that 1) one single dosage form isolated from said standardized extract comprises a total amount of major allergens which does not exceed the maximum acceptable amount of any one single major allergen of said allergenic agent and/or the concentrations of major and minor allergens in the standardized allergen extract are controlled quantitatively, and 2) that one single dosage form isolated from said standardized extract comprises an immunogenically effective amount of each of said major and optionally minor allergens.
Embodiment 2. A method for the preparation of a set comprising at least 2 mutually distinct standardized extracts of an allergenic agent, said standardized extracts each being adapted for immunization of different allergic populations that are allergic to the allergenic agent, comprising
   - obtaining an allergen containing extract derived from said allergenic agent,
   - determining the relative concentrations of allergens in said allergen containing extract,
   - on the basis of the determination, preparing a member of said set for each of at least 2 different populations allergic to the allergenic agent, each of said at least 2 different populations defining different major allergens, wherein the concentrations of said allergens in each member are adjusted so that 1) one single dosage form isolated from a member comprises a total amount of major allergens not exceeding the maximum acceptable amount of any one single major allergen of said allergenic agent and/or the concentrations of major and minor allergens in the standardized allergen extract are controlled quantitatively, and 2) that one single dosage form taken from said member comprises an immunogenically effective amount of each of said major and optionally minor allergens.
Embodiment 3. A method for preparation of standardized allergen extracts from an allergenic agent, the method comprising obtaining allergen extracts derived from the allergenic agent, determining the concentrations of major and minor allergens in the individual extracts, and if necessary adjusting the relative amounts of major and minor allergens in the extracts to obtain standardized allergen extracts where the relative amounts of major and minor allergens are within predefined boundaries so as to allow isolation, from the standardized allergen extracts, of single dosage forms wherein 1) the total amount of major allergens do not exceed the maximum acceptable amount of any one single major allergen from said allergenic agent and/or the concentrations of major and minor allergens are controlled quantitatively, and 2) the major and optionally minor allergens are present in immunologically effective amounts.
Embodiment 4. The method according to any one of the preceding embodiments, wherein multiple extracts are prepared and wherein major allergen and minor allergen concentration in any one of said multiple extracts has a variation of at most 50% compared to any other one of said multiple extracts.
Embodiment 5. The method according to embodiment Embodiment 4, wherein the variation is at most 40%, such as at most 30%, 25%, 15%, 5%, 2% and at most 1%.
Embodiment 6. The method according to any one of the preceding embodiments, wherein multiple extracts are prepared and wherein the concentration of any one major allergen and/or of any one minor allergen in any one of said multiple extracts does not exceed the concentration in any other one of said multiple extracts by a factor of more than 6.
Embodiment 7. The method according to embodiment Embodiment 6, wherein the factor does not exceed 5, such as 4, 3, and 2.
Embodiment 8. A method for inducing tolerance in a subject who is allergic to an allergenic agent, the method comprising repeated administrations of single dosage forms isolated from a standardized allergen extract comprising allergens derived from said allergenic agent, wherein the relative amounts of individual allergens in the single dosage forms are kept substantially constant over time.
Embodiment 9. The method according to embodiment Embodiment 8, wherein the single dosage forms are from an allergen extract which has been matched with either the subject's allergen sensitisation profile or the allergen sensitisation profile of an allergic population, so that 1) the total amount of allergens which constitute allergens in the subject or constitute major allergens in the allergic population does not exceed the maximum acceptable amount of any one single major allergen of said allergenic agent and/or the concentrations of major and minor allergens in the standardized allergen extract are controlled quantitatively, and 2) that one single dosage form of said allergen extract comprises an immunogenically effective amount of each of said allergens.
Embodiment 10. A method for inducing tolerance in a subject who is allergic to an allergenic agent, the method comprising obtaining the allergen sensitisation profile of said subject or of the allergenic population to which the subject belongs, selecting a standardized allergen extract or allergen composition which matches the allergens to which the subject is allergic or which matches the major allergens reactive in the allergenic population, and subsequently administering repeated single dosage forms isolated from the standardized allergen extract or allergen composition to induce tolerance to the allergenic agent, wherein the standardized allergen extract and allergen composition are ones wherein 1) one single dosage form thereof comprises a total amount of major allergens found in said population not exceeding the maximum acceptable amount of any one single major allergen of said allergenic agent and/or the concentrations of major and minor allergens in the standardized allergen extract and allergen composition are controlled quantitatively, and 2) that one single dosage form thereof comprises an immunogenically effective amount of each of said major and optionally minor allergens.
Embodiment 11. The method according to any one of embodiments Embodiment 8-Embodiment 10, wherein the standardized allergen extract is part of a set of at least 2 distinct allergen extracts wherein each distinct extract matches a population according to major allergens reactive in said population.
Embodiment 12. The method according to any one of embodiments Embodiment 8-Embodiment 11, wherein the single dosage of the allergen extract is increased over time to reach a maintenance dose.
Embodiment 13. The method according to any one of embodiments Embodiment 8-Embodiment 12, wherein the single dosage form is selected from the group consisting of an injectable form, a solid form such as a tablet, compresed or non-compressed, a capsule and a lozenge.
Embodiment 14. The method according to any one of embodiment Embodiment 8-Embodiment 13, wherein the single dosage form is administered via a route selected from the parenteral route, such as the subcutaneous route, the intracutaneous route, and the intramuscular route; the oralmucosal route, such as the oral, buccal, sublinqual, and gastrointestinal routes.
Embodiment 15. The method according to any one of the preceding embodiments, wherein the concentration of both major and minor allergens in the standardized allergen extract is controlled quantitatively to be within predefined limits.
Embodiment 16. The method according to embodiment Embodiment 15, wherein the concentration of each minor allergen in the standardized extract is lower than the concentration of the major allergen of lowest concentration in the defined extract.
Embodiment 17. The method according to embodiment Embodiment 15 or Embodiment 16, wherein the weight ratio between any minor allergen and the most abundant major allergen in a single dosage form does not exceed 1:50.
Embodiment 18. The method according to embodiment Embodiment 17, wherein the weight ratio between any minor allergen and the most abundant major allergen does not exceed 1:100.
Embodiment 19. The method according to embodiment Embodiment 17, wherein the weight ratio between any minor allergen and the most abundant major allergen does not exceed 1:200.
Embodiment 20. The method according to any one of the preceding embodiments, wherein the weight ratio between the most abundant major allergen and any other major allergen in a single dosage form does not exceed 30: 1.
Embodiment 21. The method according to embodiment Embodiment 20, wherein the weight ratio between the most abundant and any other major allergen does not exceed 15: 1.
Embodiment 22. The method according to embodiment Embodiment 20, wherein the weight ratio between the most abundant and any other major allergen does not exceed 10: 1.
Embodiment 23. The method according to embodiment Embodiment 20, wherein the weight ratio between the most abundant and any other major allergen does not exceed 5: 1.
Embodiment 24. The method according to embodiment Embodiment 20, wherein the weight ratio between the most abundant and any other major allergen does not exceed 2.5:1.
Embodiment 25. The method according to any one of the preceding embodiments, wherein said standardized allergenic extract comprises all major allergens from said allergenic agent.
Embodiment 26. A method for the preparation of a standardized extract of an allergenic agent, comprising
   - obtaining an allergen containing extract from the allergenic agent,
   - determining the relative concentrations of allergens in said allergen containing extract, wherein said allergens are defined by the allergen sensitisation profile of an allergic subject,
   - adjusting the concentrations of said allergens so that 1) one single dosage form of said extract comprises a total amount of allergens which does not exceed the maximum acceptable amount of any one single allergen of said allergenic agent and/or the concentrations of all allergens in the standardized allergen extract are controlled quantitatively, and 2) that one single dosage form of said extract comprises an immunogenically effective amount of each of said allergens.
Embodiment 27. The method according to any one of embodiments Embodiment 1-Embodiment 3 and Embodiment 15-Embodiment 26, insofar as these depend from any of embodiments Embodiment 1-Embodiment 3, which comprises the further step of concentrating or diluting the standardized extract.
Embodiment 28. A method for the preparation of a pharmaceutical composition for inducing tolerance to an allergen, the method comprising preparing a standardized extract according to any one of embodiments Embodiment 1-Embodiment 3 and Embodiment 15-Embodiment 27, insofar as these depend from any of embodiments Embodiment 1-Embodiment 3, and subsequently formulating the standardized extract together with a pharmaceutically and immunologically acceptable carrier, vehicle or diluent.
Embodiment 29. A method for preparing an allergen composition, the method comprising determining the allergen sensitisation profile of a population so as to identify major allergens and minor allergens reactive in said population, subsequently admixing the major and minor allergens thus identified, so that 1) the concentrations of said major and minor allergens in one single dosage form isolated from said allergen composition comprises a total amount of major allergens which does not exceed the maximum acceptable amount of any one single major allergen of said allergenic agent and/or the concentrations of major and minor allergens in the allergen composition are controlled quantitatively, and 2) that one single dosage form isolated from said allergen composition comprises an immunogenically effective amount of each of said major and optionally minor allergens.
Embodiment 30. The method according to embodiment Embodiment 29, wherein the relative concentrations of major and minor allergens are as defined for the standardized allergenic extracts in any one of embodiments Embodiment 15-Embodiment 24.
Embodiment 31. The method according to embodiment Embodiment 29 or Embodiment 30, wherein the allergens are isolated from a natural source and/or recombinantly produced and/or prepared by means of synthesis.
Embodiment 32. The method according to any one of embodiments Embodiment 29-Embodiment 31, which comprises the further step of concentrating or diluting the allergen composition.
Embodiment 33. A method for the preparation of a pharmaceutical composition for inducing tolerance to an allergen, the method comprising preparing an allergen composition according to the method of any one of embodiments Embodiment 29-Embodiment 32, and subsequently formulating the allergen composition together with a pharmaceutically and immunologically acceptable carrier, vehicle or diluent.
Embodiment 34. The method according to any one of the preceding embodiments, wherein the allergic population constitutes a subset of a larger population of subjects that are allergic to the allergenic agent.
Embodiment 35. The method according to any one of the preceding embodiments, wherein the allergen is selected from a pollen allergen, a fungal or mould allergen, an insect allergen, a dust mite allergen, and a food allergen.
Embodiment 36. The method according to any one of embodiments Embodiment 1-Embodiment 34, wherein the allergen, major or minor, is selected from the group consisting of Bet v 1, Bet v 2, Aln g 1, Cor a 1 and Car b 1, Cas s 1, Cas s 5, Que a 1, Cry j 1, Cry j 2 , Cup a 1, Cup s 1, Jun a 1, Jun a 2, Jun a 3, Jun o 4, Jun s 1, Jun v 1, Ole e 1, Ole e2,, Ole e 5, Ole e 8, Ole e 9, Syr v 1, Lig v 1, Pla l 1, Pla a 2, Pla a 3, Amb a 1, Amb a 2, Amb a 3, Amb a 5, Amb a 6, Amb a 7, Amb t 5, Art v 1, Art v 2, Art v 3, Art v 4, Par j 1 , Par j 2, Par j 3, Par o 1, Sal k 1, Ave e 1, Cyn d 1, Cyn d 7, Cyn d 12, Dac g 1, Dag g 2, Dag g 3, Fes p 1, Fes p 4, Hol l 1, Lol p 1. Lol p 5, Lol p 2, Lol p 3, Pha a 1, Pas n 1, Phl p 1, Phl p 2, Phl p 3, Phl p 4, Phl p 5, Phl p 6, Poa p 1, Poa p 5, Sec c 1, Sec c 5, Sor h 1, Der f 1 , Der f 2, Der f 3, Der f 7, Der f, 10, Der f 11, Der f 14, Der p 1, Der p 2, , Der p 3, Der p 4, Der p 5, Der p 6, Der p 7, Der p 8, Der p 10, Der m 1, Eur m 2, Eur m 14, Gly d 1, Gly d 2, Lep d 2, Lep d 5, Lep d 7, Lep d 10, Lep d 13, Blo t 1, Blo t 3, Blo t 6, Blo t 10, Tyr p 2, Bla g 1, Bla g 2, Bla g 4, Bla g 5, Bla g 6, Per a 1, per a 3, per a 7, Fel d 1, Fel d 2, Can f 1, Can f 2 , Can f 3, Bos d 2, Bos d 4, Bos d 8, Equ c 1, Equ c 2, Equ c 3 , Mus m 1, Rat n 1, Cav p1, Cav p 2, Apis m 1, Api m 2 ,Apis m 4, Ves v 1, Ves v 2, Ves v 5, Dol m 1, Dol m 2, Dol m 5, Pol a 1, Pol a 2, Pol a 5, Sol i 1, Sol i 2, Sol i 3 and Sol i 4, Alt a 1, Alt a 2, Alt a 3, Alt a 6, Cla h 1, Cha l 2, Cha l 3, Asp f 1, Asp f 2, Asp f 3, , Mal d 1, Gly m 1, Gly m 2, Gly m 3, Ara h 1, Ara h 2, Ara h 3, Ara h 4, and Ara h 5.
Embodiment 37. A anti-allergy kit, said kit comprising
   - diagnostic means for profiling an allergic subject so as to determine which allergens derived from an allergenic agent are allergenic in the subject,
   - a set of distinct standardized allergen extracts, wherein each of said extracts comprises allergens derived from said allergenic agent, said distinct allergen standardized allergen extracts being defined relative to allergic populations so that each distinct standardized allergen extract comprises major allergens reactive in each such population so that i) the weight ratio between the most abundant major allergen and any other major allergen reactive in the population does not exceed 30:1 and ii) the ratio between any minor allergen and the most abundant major allergen reactive in the population does not exceed 1:50 or the concentrations of minor allergens are within defined limits.
Embodiment 38. An anti-allergy kit, said kit comprising
   - diagnostic means for profiling an allergic subject so as to determine which allergens derived from an allergenic agent are allergenic in the subject,
   - a set of distinct allergen compositions, wherein each of said distinct allergen compositions comprises allergens derived from said allergenic agent, said distinct allergen compositions being defined relative to allergic populations so that each allergen composition comprises major allergens reactive in each such population so that i) the weight ratio between the most abundant major allergen and any other major allergen reactive in the population does not exceed 30:1 and ii) the ratio between any minor allergen and the most abundant major allergen does not exceed 1:50 or the concentrations of minor allergens are within defined limits.
Embodiment 39. The kit according to embodiment Embodiment 38, wherein the allergens are either isolated allergens or allergens that are recombinantly produced.
Embodiment 40. The kit according to any one of embodiments Embodiment 37-Embodiment 39, which comprises all major allergens of said allergenic agent.
Embodiment 41. The kit according to any one of embodiments Embodiment 37-Embodiment 40, wherein ratios between major and minor allergens are as defined in embodiment Embodiment 18 or Embodiment 19.
Embodiment 42. The kit according to any one of embodiments 35-39, wherein the ratios between major allergens are as defined in any one of embodiments Embodiment 19-Embodiment 24.

## Claims

1. A method for the preparation of a standardized extract of olive tree pollen allergens, comprising
- obtaining an allergen containing extract derived from said olive tree pollen,
- determining the relative concentrations of major and minor allergens in said allergen containing extract, wherein said major and minor allergens are defined according to the allergen sensitisation profiles obtained from a population allergic to olive tree pollen allergens,
- adjusting the concentrations of said major and minor allergens to obtain said standardized extract so that 1) the concentrations of major and minor allergens in the standardized allergen extract are controlled quantitatively, and 2) that one single dosage form isolated from said standardized extract comprises an immunogenically effective amount of each of said major and optionally minor allergens.

2. A method for preparation of standardized allergen extracts from olive tree pollen allergens, the method comprising obtaining allergen extracts derived from the allergenic agent, determining the concentrations of major and minor allergens in the individual extracts, and if necessary adjusting the relative amounts of major and minor allergens in the extracts to obtain standardized allergen extracts where the relative amounts of major and minor allergens are within predefined boundaries so as to allow isolation, from the standardized allergen extracts, of single dosage forms wherein 1) the concentrations of major and minor allergens are controlled quantitatively, and 2) the major and optionally minor allergens are present in immunologically effective amounts.

3. The method according to any one of claims Embodiment 1 and 2, wherein the concentration of both major and minor allergens in the standardized allergen extract is controlled quantitatively to be within predefined limits.

4. The method according to claim 3, wherein the concentration of each minor allergen in the standardized extract is lower than the concentration of the major allergen of lowest concentration in the defined extract.

5. The method according to claim Embodiment 15 or 4, wherein the weight ratio between any minor allergen and the most abundant major allergen in a single dosage form does not exceed 1:50.

6. The method according to claim Embodiment 17, wherein the weight ratio between any minor allergen and the most abundant major allergen does not exceed 1:100.

7. The method according to claim 5, wherein the weight ratio between any minor allergen and the most abundant major allergen does not exceed 1:200.

8. The method according to claim Embodiment 1, wherein the population allergic to the allergenic agent has a size of at least 30 individuals and all individuals lives in a defined geographical area.

9. The method according to claim Embodiment 1, wherein the population allergic to the allergenic agent lives in geographical areas where olive trees are extensively grown and exhibit high IgE levels to the allergen Ole e 9.

10. The method according to any one of claims Embodiment 1 to 9, wherein the total amount of major allergens do not exceed the maximum acceptable amount of any one single major allergen from said allergenic agent.

11. The method according to any one of claims Embodiment 1 or Embodiment 3, wherein the concentrations of the major and minor are controlled quantitatively by use of ELISA technique.

12. The method according to any one of claims Embodiment 1 or Embodiment 3, wherein a minor allergen is Ole e 9.

13. A pharmaceutical composition comprising a standardized extract obtained according to the method of any one of claims Embodiment 1 or 2 and a pharmaceutically and immunologically acceptable carrier, vehicle or diluent.

14. A pharmaceutical composition according to claim 13 for use in inducing tolerance to an allergen present in said standardized allergen extract in a subject.

15. A method for preparing monoclonal antibodies reactive with Ole e 9 allergen comprising providing Ole e 9 allergen purified from olive tree pollen, injecting female BALB/c mice intraperitoneally with 16 µg of said allergen in Freund's complete adjuvant, boosting with an identical amount of antigen in Freund's incomplete adjuvant on day 15 and day 30 after injection, boosting on day 42 intravenously with the same dose of antigen in PBS, fusing spleen cells from immunized mice with P3.X63.Ag8.653 myeloma cells three days later, screening cell culture supernatants ten days after the fusion for anti-Ole e 9 specific antibodies by an ELISA with the allergen on the solid phase using P3.X63.Ag8 mouse myeloma culture supernatant as a negative control, cloning positive hybridomas and subcloning by limiting dilution, and purifying selected monoclonal antibodies from hybridoma culture supernatants using a Protein G Sepharose affinity column.
